(19) Europäisches Patentamt
European Patent Office
Office européen des brevets

(11) EP 0 864 542 A2

(12) EUROPÄISCHE PATENTANMELDUNG

(43) Veröffentlichungstag:
**16.09.1998 Patentblatt 1998/38**

(51) Int. Cl.[6]: **C02F 3/34**, C02F 3/28, C12R 1/01, C12Q 1/68

(21) Anmeldenummer: **98103842.5**

(22) Anmeldetag: **04.03.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **12.03.1997 DE 19710010**

(71) Anmelder:
**Solvay Deutschland GmbH**
**D-30173 Hannover (DE)**

(72) Erfinder:
- **Diekert, Gabriele, Prof. Dr.**
  **70193 Stuttgart (DE)**
- **Wohlfarth, Gert, Dr.**
  **70372 Stuttgart (DE)**
- **Neumann, Anke**
  **70197 Stuttgart (DE)**
- **Scholz-Muramatsu, Heidrun, Dr.**
  **70569 Stuttgart (DE)**
- **Granzow, Silke**
  **71254 Ditzingen (DE)**
- **Eisenbeis, Martina**
  **73240 Wendlingen (DE)**

(74) Vertreter: **Gosmann, Martin**
**Solvay Pharmaceuticals GmbH**
**Hans-Böckler-Allee 20**
**30173 Hannover (DE)**

(54) **Verfahren zur biologischen Behandlung von Grund- oder Prozessabwässern zur Entfernung von halogenierten, ungesättigten Kohlenwasserstoffen**

(57) Beschrieben wird ein neues Verfahren zur mikrobiologischen Reinigung von Wässern, insbesondere von Grundwasser oder von Prozeßwässern, welche halogenierte, ungesättigte Kohlenwasserstoffe wie z. B. insbesondere chlorierte Ethene bzw. chlorierte Propene enthalten. Weiterhin werden im Rahmen der vorliegenden Erfindung auch zusätzliche Hilfsmittel zur effektiven Durchführung bzw. Unterstützung des mikrobiologischen Grundwasser- bzw. Prozeßabwasserreinigungsverfahrens bereitgestellt. So werden syntrophe Mischkulturen für die Dechlorierung von chlorierten Ethenen bzw. chlorierten Propenen beschrieben und auch DNA-Sequenzen, die sich als Gensonde zum Auffinden von Dehalogenase-Aktivität eignen.



Printed by Xerox (UK) Business Services
2.16.3/3.4

## Beschreibung

Die Erfindung bezieht sich auf ein neues Verfahren zur mikrobiologischen Reinigung von Wässern, insbesondere von Grundwässern oder von Prozeßabwässern, welche halogenierte, ungesättigte Kohlenwasserstoffe wie z. B. insbesondere chlorierte Ethene und/oder chlorierte Propene enthalten. Weiterhin bezieht sich die Erfindung auch auf die Bereitstellung zusätzlicher Hilfsmittel zur effektiven Durchführung bzw. Unterstützung des mikrobiologischen Grundwasser- bzw. Prozeßabwasserreinigungsverfahrens.

Synthetische chlorierte Lösemittel wie z. B. Tetrachlorethen (Perchlorethen; PCE) und z. B. Trichlorethen (TCE) werden in vielen verschiedenen industriellen Verfahren wegen ihrer günstigen anwendungstechnischen Eigenschaften eingesetzt; beispielsweise sind diese Verbindungen im Gegensatz zu den unchlorierten Verbindungen schwer entflammbar und nicht explosionsgefährlich. Durch sorglosen Umgang, Lagerung und Entsorgung sowie durch die hohe chemische Stabilität dieser Verbindungen gehören diese heute allerdings zu den häufigsten Verunreinigungen des Grundwassers. Die Anwesenheit von PCE und TCE im Grundwasser bedarf besonderer Beachtung, insbesondere wegen der potentiell toxischen und kanzerogenen Wirkungen dieser Substanzen. Die Entfernung von PCE und TCE ist daher entscheidend für die Gewährleistung einer hohen Qualität des Grundwassers, welches eine wichtige Quelle für Trinkwasser darstellt. Im Stand der Technik wurden bereits einige Anstrengungen unternommen, um das Problem der PCE- und TCE-Verunreinigungen im Grundwasser zu beheben. So werden Grundwässer, die mit chlorierten Ethenen kontaminiert sind, zur Zeit überwiegend durch Aktivkohleadsorption gereinigt. Die dabei entstehende CKW-beladene Aktivkohle (CKW = Chlorkohlenwasserstoff) muß jedoch als Sondermüll entsorgt werden, was derzeit durch die Verbrennung in Sondermüllverbrennungsanlagen bewerkstelligt wird. Die Reinigung von kontaminierten Grundwässern über Aktivkohle ist jedoch in vielen Fällen unökonomisch, im Hinblick auf die Entsorgung der CKW-beladenen Aktivkohle ebenfalls problematisch und bei einigen Grundwässern sogar nicht anwendbar. So ist beispielsweise die Herstellung der Aktivkohle mit beträchtlichen Energiekosten verbunden; beim Transport vom Ort der Kontamination zum Ort der Entsorgung sind Schadstoffemissionen nicht auszuschließen; bei Mischkontaminationen können z. B. Chromatographieeffekte auftreten, d. h. eine Entfernung der Verunreinigungen gelingt dann nicht; oder bei kalk- und/oder eisenhaltigen Grundwässern besteht eine Verstopfungsgefahr der Aktivkohleadsorber durch Eisenhydroxid- bzw. Kalkablagerungen.

Neben diesen physikalisch/chemischen Verfahren gibt es bereits auch Vorschläge für biologische Verfahren zur Reinigung von CKW-belasteten Grundwässern. Beispielsweise wurden im sogenannten "Eppelheim-Verfahren" CKW/BTX-kontaminierte Grundwässer (BTX = Benzol-, Toluol-, Xylol-Derivate) in einem dreistufigen "on-site"-Prozeß biologisch gereinigt, indem das Grundwasser nacheinander denitrifizierenden, anaeroben und aeroben Bedingungen ausgesetzt wurde. Dabei wurde für den Abbau der Schadstoffe die standorteigene Mikroflora des kontaminierten Bodens genutzt. Über die Zusammensetzung dieser Flora und ihre physiologischen Eigenschaften ist derzeit jedoch nichts bekannt. In Unkenntnis der Wirkungsweise der Mikroflora ist es jedoch nur schwer möglich, den Reinigungsprozeß zu optimieren bzw. Betriebsstörungen zu vermeiden oder zu beheben. Auch ist die Übertragbarkeit der "on-site" gewonnenen Erkenntnisse auf andere CKW-kontaminierte Standorte nicht immer in wünschenswerter Weise gegeben, so daß oftmals ortsansässige Mikroflora mit dehalogenierenden Eigenschaften aufgefunden und optimiert werden muß.

Die der Erfindung zugrundeliegende mikrobiologische Problematik soll am Beispiel der Tetrachlorethen-Dechlorierung, insbesondere in Grundwasser, kurz erläutert werden. Da Tetrachlorethen, auch Perchlorethen (PCE) genannt, einem aeroben Bioabbau widersteht, kommt für den biologischen "Abbau", d. h. für die Dechlorierung bzw. für die Reduktion von PCE lediglich ein anaerobes Verfahren bzw. Teilverfahren in Betracht. Hierbei ist bereits bekannt, daß PCE-kontaminiertes Grundwasser in einem biotechnologischen Prozeß durch reduktive Dechlorierung von Perchlorethen (PCE) unter Bildung von Ethen gereinigt werden kann. Diese Dechlorierung von PCE zu Ethen erfolgt in dem bekannten biotechnologischen Prozeß unter Verwendung des anaeroben Bakteriums Dehalospirillum multivorans, wobei das PCE zunächst zu Trichlorethen (TCE) und sodann das TCE weiter zum cis-1,2-Dichlorethen (DCE) reduziert wird. Die weitere Dechlorierung erfolgt in diesem Verfahren des Standes der Technik durch eine hochangereicherte anaerobe Mischkultur, die frei von methanogenen Bakterien ist, aber deren weitere Zusammensetzung unbekannt ist. Diese hochangereicherte anaerobe Mischkultur reduziert DCE über Vinylchlorid (VC) zum Endprodukt Ethen, ohne daß eine nennenswerte Akkumulation von VC auftritt; siehe z. B. Literatur [7].

In den mikrobiologischen Verfahren des Standes der Technik erfolgt die PCE-Reduktion somit quasi in zwei Stufen:

1. Stufe: PCE -------> TCE -------> DCE
2. Stufe: DCE -------> (VC) -------> Ethen.

Es bestand die Aufgabe, die noch nicht optimal arbeitenden mikrobiologischen Verfahren des Standes der Technik für die Grundwasserreinigung zu optimieren und auch für die Reinigung von Prozeßabwässern nutzbar zu machen und weitere geeignete Hilfsmittel zur Unterstützung und für die Anwendung des Verfahrens bereitzustellen.

Die Aufgabe wird gelöst durch die in den Ansprüchen angegebenen Ausgestaltungen der Erfindung.

Die Erfindung betrifft daher zunächst ein Verfahren zur mikrobiologischen Reinigung von Wässern, die mit chlorierten Ethenen und/oder chlorierten Propenen kontaminiert sind, wobei sich das Verfahren dadurch auszeichnet, daß man die mit chlorierten Ethenen und/oder chlorierten Propenen belasteten Wässer, vorzugsweise Grundwässer oder Prozeßabwässer, unter Zusatz einer ausreichenden Menge einer Elektronendonor-Substanz über einen Bioreaktor leitet, der eine definierte, an einen Träger immobilisierte, syntrophe Mischkultur enthält, die aus wenigstens einem dehalogenierenden Bakterium und wenigstens einem wasserstoffbildenden strikt anaeroben Mikroorganismus besteht, wobei der wasserstoffbildende strikt anaerobe Mikroorganismus vorzugsweise gleichzeitig eine C-Quelle für das dehalogenierende Bakterium liefert.

Als wasserstoffbildender strikt anaerober Mikroorganismus kann im erfindungsgemäßen Verfahren jeder geeignete wasserstoffbildende strikt anaerobe Mikroorganismus eingesetzt werden, der mit dem dehalogenierenden Bakterium kompatibel ist. Zweckmäßiger Weise wird ein sulfidogenes Bakterium, insbesondere ein sulfatreduzierendes Bakterium, eingesetzt. Besonders bevorzugt ist hierbei als sulfatreduzierendes Bakterium Desulfovibrio desulfuricans.

Als Substrat für den strikt anaeroben wasserstoffbildenden Mikroorganismus sind an sich alle durch den jeweiligen Mikroorganismus verstoffwechselbaren Substrate geeignet, die gleichzeitig als Elektronendonor-Substanz wirken können. Zweckmäßiger Weise wird im erfindungsgemäßen Verfahren daher als Elektronendonor-Substanz Ethanol eingesetzt. Besonders vorteilhaft ist Ethanol insbesondere dann, wenn als sulfatreduzierendes Bakterium Desulfovibrio desulfuricans eingesetzt wird.

Im erfindungsgemäßen Verfahren können an sich beliebige dehalogenierende Bakterien eingesetzt werden. Insbesondere kann das dehalogenierende Bakterium (im folgenden "Dehalogenierer" genannt) ein Dehalospirillum multivorans, ein Desulfitobacterium Stamm PCE-S, oder eine hochangereicherte anaerobe Mischkultur, die Dichlorethen (DCE) zu Ethen reduzieren kann, sein. Die vorstehenden Dehalogenierer sind in den Beispielen näher beschrieben und charakterisiert.

Im Rahmen der syntrophen Verfahrensweise zum mikrobiologischen Abbau von chlorierten Ethenen und/oder chlorierten Propenen in Grundwässern bzw. Prozeßabwässern wird die zugesetzte Elektronendonor-Substanz zunächst vom wasserstoffbildenden strikt anaeroben Mikroorganismus unter Bereitstellung von Reduktionsäquivalenten für das dehalogenierende Bakterium verstoffwechselt. Der wasserstoffbildende strikt anaerobe Mikroorganismus stellt somit aus dem Elektronendonor-Substrat die Elektronen für die Reduktion der chlorierten Ethene bereit, die dann durch das dehalogenierende Bakterium auf die abzubauende Chlorethen- bzw. Chlorpropenverbindung übertragen werden. In einer bevorzugten Variante des erfindungsgemäßen Verfahrens stellt der wasserstoffbildende strikt anaerobe Mikroorganismus nicht nur die Reduktionsäquivalente für den Dehalogenierer bereit, sondern aus dem Elektronendonor-Substrat wird bei dessen Verstoffwechselung vorzugsweise gleichzeitig auch eine für den Dehalogenierer günstige C-Quelle (Kohlenstoffquelle) gebildet. Beispielsweise wird in einer bevorzugten Verfahrensweise als wasserstoffbildender strikt anaerober Mikroorganismus das sulfatreduzierende Bakterium Desulfovibrio desulfuricans und als Elektronendonor-Substanz Ethanol eingesetzt, wobei neben Wasserstoff für den Dehalogenierer auch Acetat als C-Quelle für den Dehalogenierer durch das Bakterium Desulfovibrio desulfuricans bereitgestellt wird. Durch dieses Zusammenwirken der Mikroorganismen, d. h. des wasserstoffbildenden strikt anaeroben Mikroorganismus und des Dehalogenierers, werden günstige Voraussetzungen zum wirkungsvollen Abbau von chlorierten Ethenen und/oder chlorierten Propenen geschaffen. Das Verfahren läßt sich in einfacher Weise durch die Zugabe der Elektronendonor-Substanz steuern, die sowohl die Reduktionsäquivalente für den Dehalogenierer als auch die C-Quelle für diesen bereitstellt. Die Mischkultur aus dem dehalogenierenden Bakterium und dem wasserstoffbildenden strikt anaeroben Mikroorganismus wird daher im Rahmen der Erfindung auch als syntroph bezeichnet, um das Zusammenwirken der beiden Mikroorganismen bei der Verstoffwechselung der bereitgestellten Elektronendonor-Substanz einerseits und die Reduktion der chlorierten Ethene und/oder chlorierten Propene andererseits zu umschreiben. Analog wird die vorgestellte Verfahrensweise auch als syntrophes Verfahren zum Abbau von chlorierten Ethenen und/oder chlorierten Propenen bzw. als syntrophe Dechlorierung dieser Substanzen bezeichnet.

Als Dehalogenierer kommen im Rahmen der Erfindung wie vorstehend bereits beschrieben an sich beliebige dehalogenierende Bakterien in Frage, die als Reinkultur oder als Mischkultur auf wenigstens einer der beschriebenen Stufen der Dechlorierung von perchloriertem Ethen bzw. Propen bis hin zum enthalogenierten Ethen bzw. Propen wirken können. Erfindungsgemäß ist es daher möglich, jede Teilstufe der Dechlorierung als syntrophe Verfahrensweise auszugestalten. So kann beispielsweise die eingangs beispielhaft beschriebene Stufe 1, d. h. die Dechlorierung von PCE bis zum DCE, mit den vorstehend als bevorzugt beschriebenen Dehalogenierern betrieben werden. Ebenso kann auch die zweite obenbeschriebene Verfahrensstufe, d. h. der Abbau des DCE zum Ethen unter Verwendung einer hierfür typischerweise im Stand der Technik eingesetzten hochangereicherten anaeroben Mischkultur in erfindungsgemäßer Weise syntroph durchgeführt werden. Darüber hinaus ist es auch möglich, die beiden Stufen erfindungsgemäß in einem syntrophen Gesamtverfahren zu vereinigen, d. h., das syntrophe Verfahren wird dann mit einer Mischkultur in einem Reaktor durchgeführt, die aus einem PCE dehalogenierenden Bakterium (Stufe 1), einer hochangereicherten anaeroben Mischkultur, die das entstehende DCE reduziert, und dem wasserstoffbildenden strikt anaeroben Mikroorganismus besteht.

Als Bioreaktoren zur Aufnahme der Mikroorganismen können an sich übliche Bioreaktoren verwendet werden, z. B. Festbett-, Schwebebett- oder insbesondere Fließbettreaktoren mit natürlichen oder künstlichen Aufwuchskörpern (Träger zur Immobilisierung der Biomasse) verwendet werden. Typische Träger können z. B. Sand, Sinterglas, Kunststoffgerüststrukturen, Aluminiumoxid, Keramik, Bims und andere natürliche oder künstliche Materialien sein, die zum Aufwachsen einer mikrobiellen Biomasse geeignet sind. In einer bevorzugten Ausgestaltung der Erfindung wird das Verfahren in einem Bioreaktor durchgeführt, der ein Schlaufenreaktor mit Wirbelbett ist. In typischer Verfahrensweise wird hierbei das zu behandelnde Wasser, d. h. das Grundwasser oder das Prozeßabwasser nach Reduktion von Sauerstoff, Nitrat, Eisen(III+) und Mangan(IV+) etc. über den eigentlichen Bioreaktor zum syntrophen Abbau der chlorierten Ethene und/oder chlorierten Propene geführt. In diesem Reaktor findet dann die syntrophe Dechlorierung statt, wobei das entsprechende Grundwasser bzw. Prozeßwasser über diesen Reaktor gewunschtenfalls über einige Zeit im Kreis geführt werden kann und sodann kontinuierlich eine bestimmte Menge des behandelten Grundwassers bzw. Prozeßwassers als gereinigt abgezogen werden kann. Weitere Einzelheiten zur Durchführung des erfindungsgemäßen Verfahrens sind in den Beispielen beschrieben.

Die Erfindung betrifft neben dem syntrophen Verfahren auch eine neue vorteilhafte syntrophe Mischkultur bestehend aus a) einem Dehalogenierer, der aus der Gruppe Dehalospirillum multivorans, Desulfitobacterium Stamm PCE-S und einer hochangereicherten anaeroben Mischkultur, die Dichlorethen zu Ethen reduziert, ausgewählt ist; und b) dem wasserstoffbildenden strikt anaeroben Mikroorganismus Desulfovibrio desulfuricans. Die syntrophe Kombination des Desulfovibrio desulfuricans mit einem der angegebenen Dehalogenierer erweist sich für den Abbau von chlorierten Ethenen und/oder chlorierten Propenen als besonders vorteilhaft.

Im Rahmen der Erfindung wurde in einem weiteren Teilaspekt auch gefunden, daß für die Dechlorierung von PCE bis zum DCE ein neuer Mikroorganismus vorteilhaft ist, der sowohl in konventioneller Verfahrensweise für die Dechlorierung von PCE zum DCE als auch in der hier beschriebenen erfindungsgemäßen syntrophen Verfahrensweise eingesetzt werden kann. Die Erfindung betrifft daher auch dieses neue Desulfitobacterium Stamm PCE-S, welches erhältlich ist durch Isolierung als Reinkultur aus einer angereicherten Mischkultur aus einer Bodenprobe der ehemaligen Mülldeponie Eppelheim, indem man mit der Hungate-Technik unter Schutzgas (90 % $N_2$/10 % $CO_2$) auf Agar in Gegenwart einer Elektronendonor-Substanz und von PCE als Substrat Kolonien aus einem Inoculum der "Eppelheim-Mischkultur" zieht, wobei das Inoculum zuvor in einem Batchverfahren über einige Generationen mit hohen PCE-Konzentrationen und Pyruvat gefüttert wurde; und wobei das Desulfitobacterium Stamm PCE-S durch die in den Beispielen (Tabellen 1 und 2) angegebenen Eigenschaften bzw. Merkmale charakterisiert ist.

Mit dem erfindungsgemäßen biotechnologischen Verfahren zur Reinigung von Grundwässern bzw. Prozeßabwässern, die mit chlorierten Ethenen kontaminiert sind, wird eine günstige Verfahrensweise zur Verfügung gestellt, mit der in einem anaeroben Bioreaktor on-site PCE-Kontaminationen beseitigt werden können. Die Anlaufzeit des biologischen Prozesses läßt sich verkürzen, da durch die Erfindung auch definierte Starterkulturen (z. B. in immobilisierter Form) bereitgestellt werden. Mit der Erfindung werden deutliche Verbesserungen gegenüber dem Stand der Technik erreicht. Das neue biotechnologische Verfahren hat z. B. gegenüber den physikalischen Aktivkohleadsorber-Verfahren sowie auch gegenüber dem biologischen "Eppelheim-Verfahren" eine Reihe von Vorteilen. Durch die on-site-Elimination der Schadstoffe und die Unabhängigkeit des Verfahrens vom Standort findet keine Verlagerung der Schadstoffe (z. B. auf Aktivkohle oder in die Luft) statt. Das Verfahren kann unabhängig von der vorhandenen Mikroflora an beliebigen Standorten eingesetzt werden, beispielsweise mit bereits in einem mobilen Bioreaktor etablierten syntrophen, dechlorierenden Bakterienkultur. Durch Animpfen mit speziellen Kulturen und dem Betrieb der Reaktoren unter selektiven, leicht steuerbaren Bedingungen ist die Anlaufphase des biologischen Prozesses kurz. Der Abbauprozeß kann gut kontrolliert werden und durch den Einsatz selektiver Cosubstrate (also über die Zufuhr der Elektronendonor-Substanz, die vom wasserstoffbildenden strikt anaeroben Mikroorganismus in Reduktionsäquivalente und eine Kohlenstoff-Quelle überführt wird) kann die Produktion von störendem Überschußschlamm vermieden werden. Das erfindungsgemäße Verfahren arbeitet selbst unter dem Einfluß von kalk- und eisenhaltigem Wasser in vorteilhafter Weise. Die erfindungsgemäße biologische CKW-Elemination wird weder durch Kalk noch durch Eisen gestört. Die Anwesenheit von Kalk ist sogar vorteilhaft, da beim biologischen Abbau von CKW Protonen freiwerden, die durch den anwesenden Kalk abgepuffert werden können.

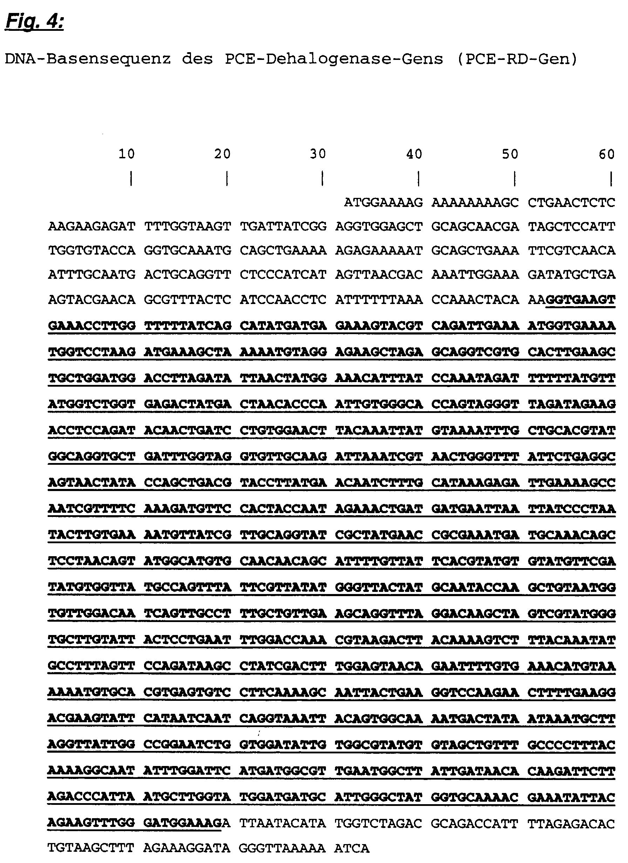

Ein weiterer Aspekt der Erfindung beschäftigt sich mit dem Problem, im Hinblik auf eine in-situ-Reinigung von Grundwasser zu prüfen, ob bereits eine leistungsfähige autochthone PCE-dechlorierende Mikroflora vorhanden ist. Hierfür muß jedoch eine geeignete Methode und entsprechende Testmittel zur Verfügung stehen, um vor Ort vorhandene, das Dehalogenase-Gen enthaltende Bakterien feststellen zu können. Weiterhin ist es auch erforderlich, im Falle von Prozeßstörungen bzw. im Falle von Anlaufschwierigkeiten bei der mikrobiologischen Reinigung von Prozeßwässern prüfen zu können, ob im Reaktor ein Bakterium mit dem Dehalogenase-Gen, also eine potentielle Dehalogenase-Aktivität, vorhanden ist, z. B. falls diese eventuell durch begeleitende Einflüsse gehemmt ist. Die Erfindung betrifft in diesem Aspekt daher ein Verfahren zum Nachweis und/oder zur Kontrolle potentiell vor Ort (Untersuchungs- bzw. Behandlungsort) vorhandener Bakterien, die das Dehalogenase-Gen, insbesondere das Gen der Dehalogenase von Dehalospirillum multivorans, enthalten, vorzugsweise in einem PCE-kontaminierten Boden- und/oder Grundwasserbereich oder in einem Bioreaktor mit immobilisiertem Zellmaterial. Bei diesem erfindungsgemäßen Verfahren isoliert man genetisches Material aus dem zu untersuchenden Boden- und/oder Grundwassermaterial oder aus dem zu kontrollierenden Bioreaktor, das danach in konventioneller Weise mit einer zu der in Fig. 4 angegebenen, wenigstens 80 % homologen DNA-Sequenz für das Dehalogenase-Gen aus Dehalospirillum multivorans oder mit wenigstens einer 0,2 kB-großen Teilsequenz davon in an sich üblicher Weise hybridisiert wird, und wobei man nachfolgend den im positiven Falle gebildeten, hybridisierten DNA-Strang in an sich üblicher Weise weiterverarbeitet und identifiziert.

Für das vorstehende Verfahren zum Nachweis und/oder zur Kontrolle von potentiell vor Ort vorhandenen Bakterien mit Dehalogenase-Gen ist daher eine DNA-Sequenz erforderlich, mit welcher auf das entsprechende Dehalogenase-Gen geprüft werden kann. Die Erfindung betrifft daher auch eine solche DNA-Sequenz mit wenigstens 80 %, vorzugsweise 90 % und insbesondere 95 % Homologie zu der in Fig. 4 angegebenen DNA-Sequenz für das Dehalogenase-Gen aus Dehalospirillum multivorans. Mit dieser Sequenz oder den vorstehend genannten Teilsequenzen als "Gensonde" bzw. "Meßsonde" läßt sich in bequemer Weise feststellen, ob Dehalogenase-Aktivität zwar potentiell vorhanden ist, aber durch Hemmung, z. B. aufgrund von bestimmten Randbedingungen, nur nicht nach außen, d. h. durch Abbau von chlorierten Ethenen bzw. chlorierten Propenen, in Erscheinung tritt.

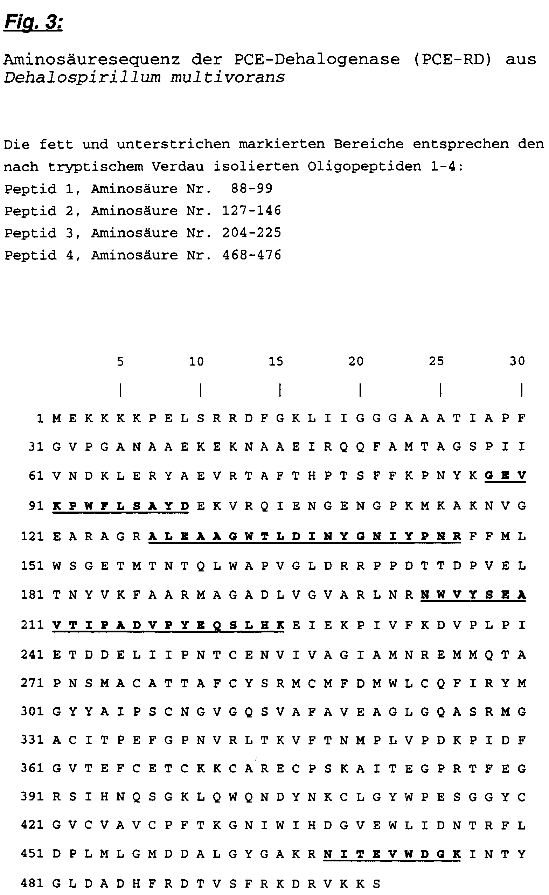

Zur Isolierung der entsprechenden DNA-Gensequenz wurde die PCE-Dehalogenase aus Dehalospirillum multivorans mit Trypsin gespalten und die Aminosäure-Sequenz von vier internen Peptiden bestimmt. Aus den vier internen Peptidsequenzen wurden die Oligonukleotid-Primer zur Klonierung des Enzyms mit Hilfe der PCR-Methode erstellt. Vom Gen der PCE-Dehalogenase (1,8 kB) wurde hierbei ein PCR-Produkt in der Größe von 1,2 kB erhalten, in den Vektor pBluescript eingebaut und anschließend in E. coli kloniert. Das PCR-Produkt wurde von beiden Seiten ansequenziert und die sequenzierten vier Referenzpeptide konnten an den in Fig. 3 markierten Stellen wiedergefunden werden. Anschließend wurde in an sich üblicher Weise das Gen der PCE-Dehalogenase aus Dehalospirillum multivorans aus einer Genbank kloniert und vollständig in an sich üblicher Weise sequenziert.

Mit der Bereitstellung des PCE-Dehalogenase-Gens aus Dehalospirillum multivorans wird ein vorteilhaftes Detektionsmittel zur Überprüfung der Anwesenheit einer potentiellen Dehalogenase-Aktivität bereitgestellt. Ebenso können auch sinnvolle Teilsequenzen mit einer Mindestgröße von 0,2 kB dieses Dehalogenase-Gens wie vorstehend beschrieben als Detektionsmaterial (Gensonde) verwendet werden. Solche Teilsequenzen können auch Teile des N- oder C-Terminus oder des "Membranankers" sein. Darüber hinaus wird durch die Bereitstellung des Gens der PCE-Dehalogenase die Möglichkeit gegeben, das Enzym in großen Mengen zu exprimieren und hiermit noch leistungsfähigere und unempfindlichere Stamme für die Sanierung von PCE-belasteten Standorten, Grundwässern und die Reinigung von Prozeßabwässern zur Verfügung zu stellen.

Die Erfindung wird beispielhaft durch die nachfolgenden Figuren sowie die nachfolgenden Beispiele näher erläutert, ohne sie jedoch in ihrem Umfang zu beschränken. Die nachfolgend angegebenen Literaturzitate dienen zur weiteren Erläuterung der in den Beispielen verwendeten allgemeinen Methoden und Arbeitsweisen.

## Literatur

In den nachfolgenden Beispielen wird auf folgende Literatur Bezug genommen:

### Beispiele 1 - 8:

[1] Holliger C., Schraa G., Stams AJM., Zehnder AJB. (1993). A highly purified enrichment culture couples the reductive dechlorination of tetrachloroethene to growth. Appl. Environ. Microbiol. **59**: 2991-1997

[2] Bauer-Kreisel P., Eisenbeis M., Scholz-Muramatsu H. (1996). Quantification of Dehalospirillum multivorans in mixed culture biofilms with an enzyme-linked immunosorbent assay. Appl. Environ. Microbiol. **62**(8): 3050-3052

[3] Christiansen N., Ahring BK. (1996).
Desulfitobacterium hafniense sp. nov., an anaerobic, reductively dechlorinating bacterium. Int. J. Syst. Bac. **46:** 442-448

[4] Neumann A., Scholz-Muramatsu H., Diekert G. (1994). Tetrachloroethene metabolism of Dehalospirillum multivorans.
Arch. Microbiol. **162:** 295-301

[5] Scholz-Muramatsu H., Neumann A., Meßmer M., Moore E., Diekert G. (1995).
Isolation and characterization of Dehalospirillum multivorans gen. nov., sp. nov., a tetrachloroethene-utilizing, strictly anaerobic bacterium.

Arch. Microbiol. **163:** 48-56

[6] Jagnow G., Haider K., Ellwardt P.-Chr. (1977).
Anaerobic dechlorination and degradation of hexachlorocyclohexane isomers by anaerobic and facultative anaerobic bacteria.
Arch. Microbiol. **115:** 285-292

[7] Granzow S., Eisenbeis M., Windfuhr C., Bauer-Kreisel P., Scholz-Muramatsu H., (1996):
Entwicklung eines Biofilmreaktors zur vollständigen Dechlorierung von Tetrachlorethen mit Dehalospirillum multivorans und einer Dichlorethen-dechlorierenden Mischkultur als Starterkulturen für die vollständige Dechlorierung von Tetrachlorethen. In: Proceedings zur Dechema-Jahrestagung '96, 21.-23.05.1996, Wiesbaden, Band I, Seite 446.

**Beispiel 9:**

[8] A. Neumann, G. Wohlfarth, G. Diekert (1996).
Purification and characterization of tetrachlorethene reductive dehalogenase from Dehalospirillum multivorans. J. Biol. Chem. 271: 16515-16519

[9] K. L. Stone, H. B. LoPresti, J. H. Crawford, R. DeAngelis., K. R. Williams (1989).
Enzymatic digestion of proteins and HPLC peptide isolation, S. 31 - 47. In: P. T. Matsudaira (Hrsg.), A practical guide to protein and peptide purification for micorsequencing. Academic Press, USA

[10] F. M. Ausubel, R. Brent, R. E. Kingston, D. D. Moore, J. G. Seidmann, J. A. Smith, K. Struhl (1994 - 1997). Current protocols in molecular biology. John Wiley & Sons, USA.

## Erläuterungen zu den Figuren

**Figur 1:**
Grundschema des erfindungsgemäßen Verfahrens zur vollständigen reduktiven Dechlorierung von chlorierten Ethenen aus kontaminiertem Grundwasser bzw. Prozeßabwasser; mit fakultativem, vorgeschaltetem Reaktor zur Eliminierung von Sauerstoff, Nitrat, Eisen(II+), Mangan(IV+).

**Figur 2:**
Abbauleistung einer DCE abbauenden Mischkultur (Apparenter $V_{max1}$ und $V_{max2)}$

**Figur 3:**
Aminosäurensequenz der PCE-Dehalogenase (PCE-RD) aus Dehalospirillum multivorans

**Figur 4:**
DNA-Basensequenz des PCE-Dehalogenase-Gens (PCE-RD-Gen) aus Dehalospirillum multivorans

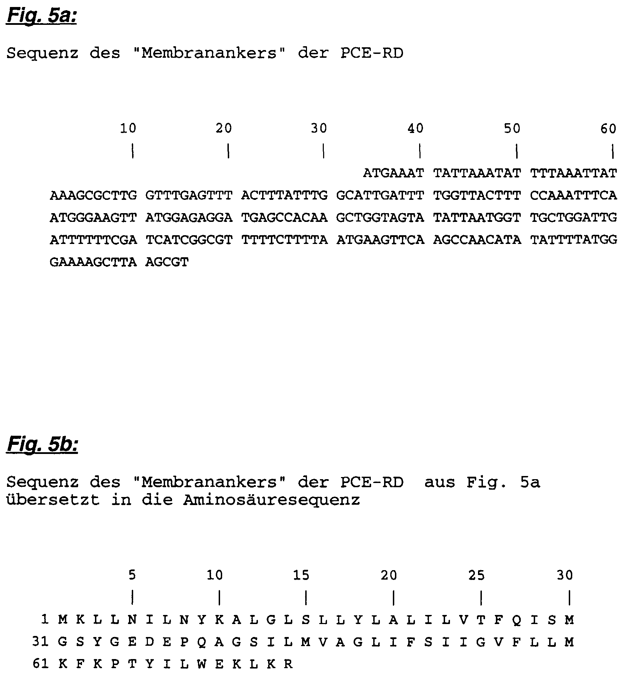

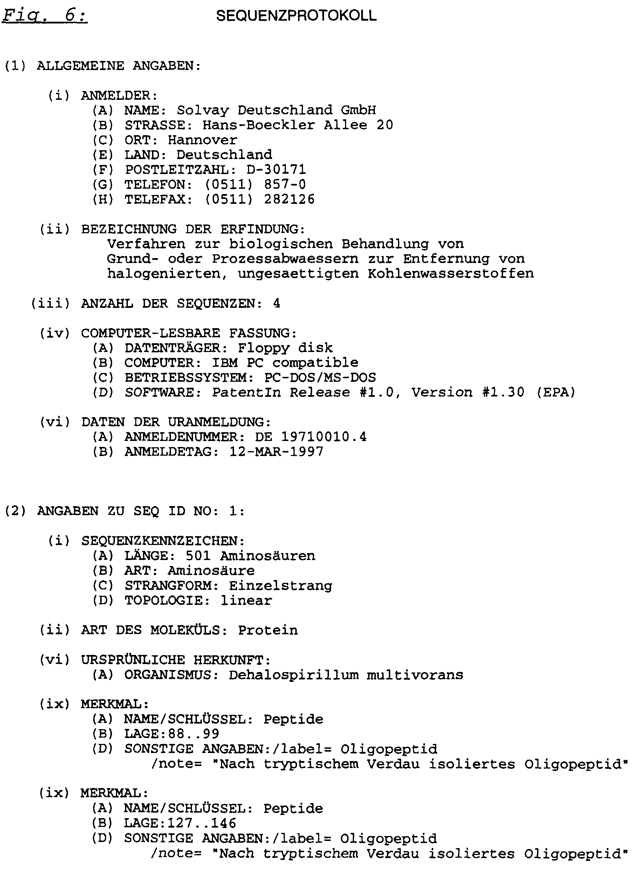

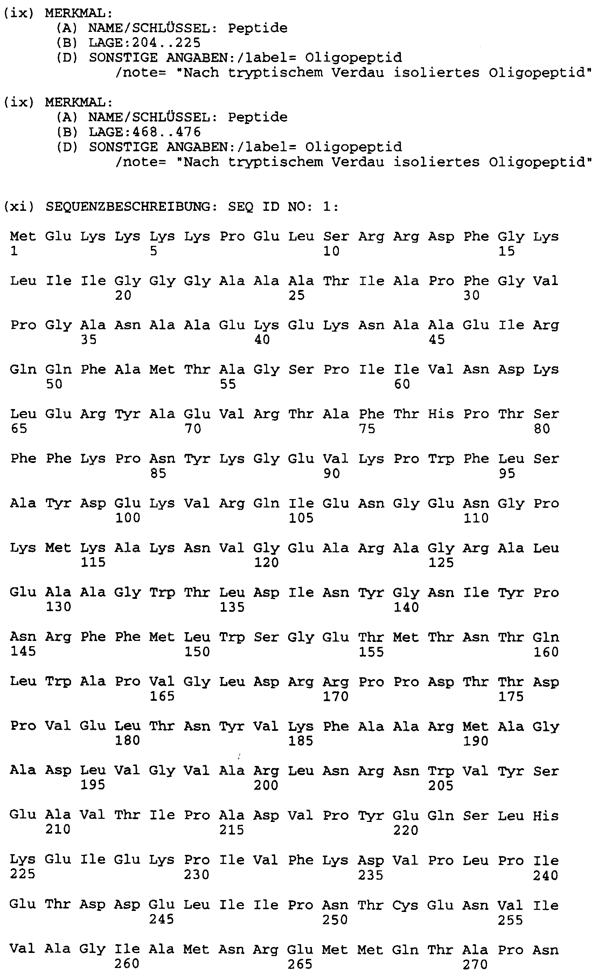

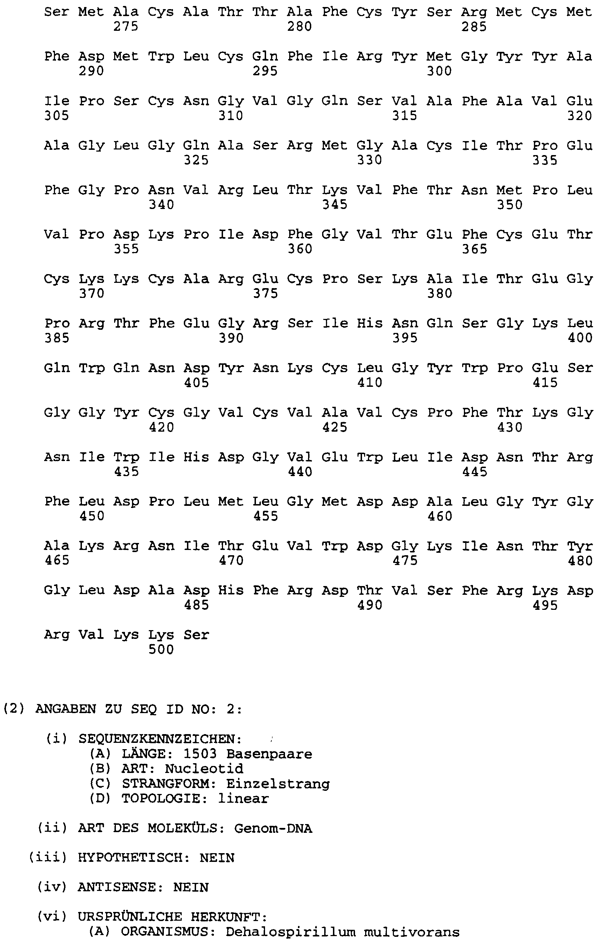

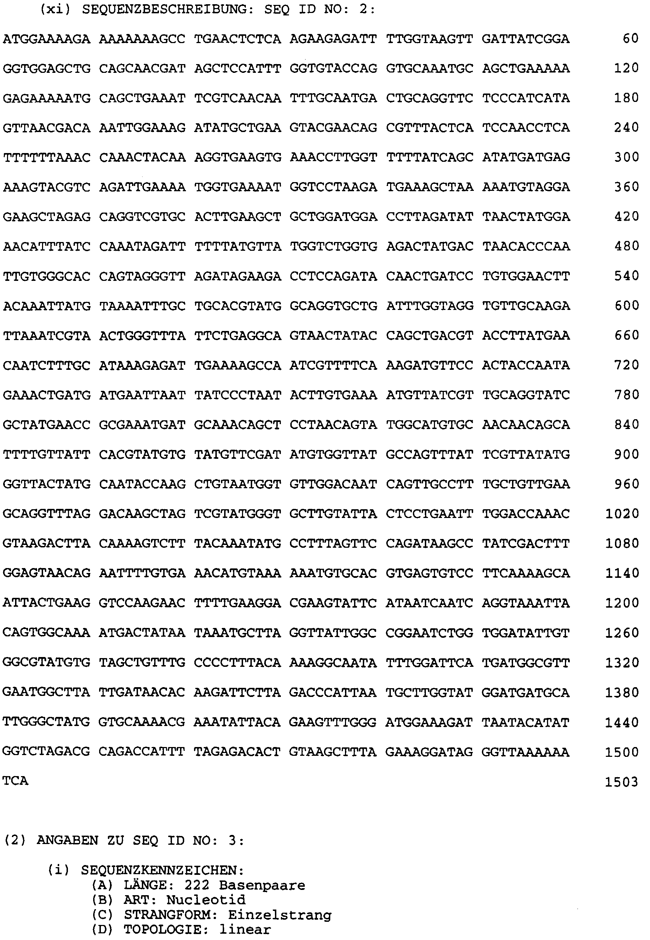

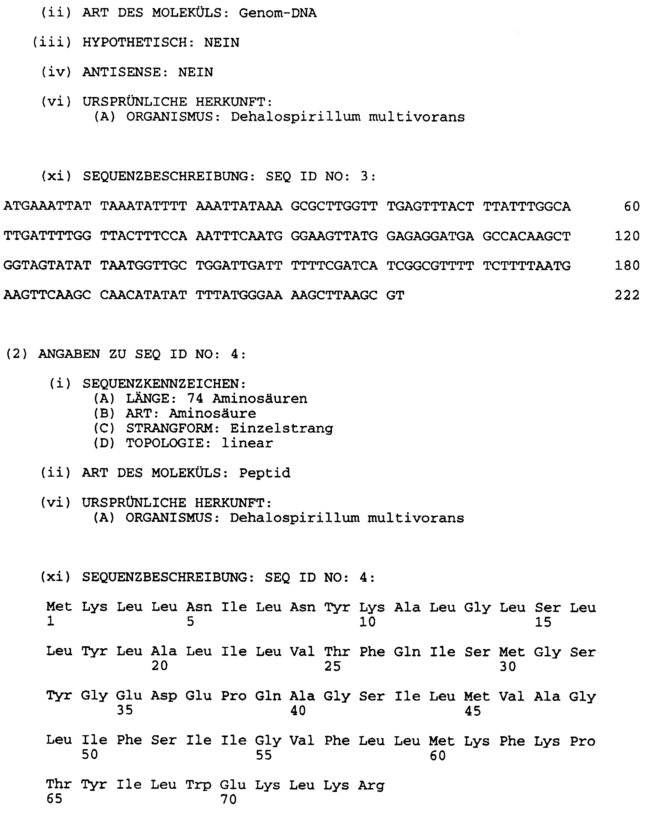

**Figur 5:**
DNA-Sequenz des "Membranankers" der PCE-RD (Fig. 5a) und die Übersetzung dieser DNA-Sequenz in die Aminosäurensequenz (Fig. 5b)

**Figur 6:**
Sequenzprotokolle zu den in den Figuren 3 bis 5 angegebenen Sequenzen.

## Beispiele

**Beispiel 1:**

Syntrophe Dechlonierung von PCE-Kontaminationen in Wasser zu DCE

Der Abbau von PCE-Kontaminationen in Wasser über TCE bis zum DCE wurde als syntrophe Dechlorierung unter folgenden Bedingungen und Variationen ausgeführt:

Syntrophe Mikroorganismen-Kombination:

A) ein wasserstoffbildendes strikt anaerobes Bakterium, das gleichzeitig Acetat als Kohlenstoffquelle für den dehalogenierenden Mikroorganismus bildet;

<u>A1:</u> Desulfovibrio desulfuricans
<u>A2:</u> Syntrophobacter wolinii
<u>A3:</u> Saccharolytische Clostridien, z. B. C. pasteurianum.

B) ein PCE-dehalogenierendes Bakterium (Produktion von cis-1,2-Dichlorethen (DCE) aus Tetrachlorethen (PCE)):

<u>B1:</u> Dehalospirillum multivorans
(siehe Neumann A., Scholz-Muramatsu H., Diekert G. 1994). Tetrachloroethene metabolism of Dehalospirillum multivorans. Arch. Microbiol. 162: 295-301 oder Lit. [5])

<u>B2:</u> Desulfitobacterium Stamm PCE-S wie im Beispiel 4 isoliert und im Beispiel 5 charakterisiert.
(siehe Christiansen N., Ahring BK. (1996). Desulfitobacterium hafniense sp. nov., an Anaerobic, reductively dechlorinating bacterium. Int. J. Syst. Bac. 46: 442-448)

Anwendungsbereich: Teilreinigung von Wasser, kontaminiert durch PCE und TCE, zu DCE

Reaktorform: wahlweise Festbett-, Schwebebett-, Wirbelschichtreaktor

Aufwuchsmaterial: wahlweise Kunststoff, Aluminiumoxid, Keramik, Sinterglas, Quarzsand, Bims

Prozeßführung: Einstufiger anaerober Prozeß

Es wurden folgende Umsatzraten der Bakterien im Batchversuch gemessen:

mit Ethanol als Substrat: (siehe Tabellen 11 und 12) Desulfovibrio desulfuricans + D. multivorans 800 - 900 nmol $Cl^-$/min/mg Zellprotein (Chloridfreisetzung aus PCE)
mit Propionsäure als Substrat: (siehe Tabellen 11 und 12) Syntrophobacter wolinii + D. multivorans 420 nmol $Cl^-$/min/mg Zellprotein (Chloridfreisetzung aus PCE).

**<u>Beispiel 2:</u>**

Syntrophe Dechlorierung von DCE in Wasser zu Ethen

Der weitere Abbau des in Beispiel 1 als Abbauprodukt von PCE gebildeten DCE zum Ethen wurde als syntrophe Dechlorierung unter folgenden Bedingungen und Variationen ausgeführt:

Syntrophe Mikroorganismen-Kombination:

A) ein wasserstoffbildendes strikt anaerobes Bakterium, das gleichzeitig Acetat als Kohlenstoffquelle für den dehalogenierenden Mikroorganismus bildet:

<u>A1:</u> Desulfovibrio desulfuricans
<u>A2:</u> Syntrophobacter wolinii
<u>A3:</u> Saccharolytische Clostridien, z. B. C. pasteurianum

C) hochangereicherte Mischkultur aus 3 bis 4 verschiedenen Bakterienarten mit der Fähigkeit zur Dechlorierung von cis-1,2-Dichlorethen (DCE) über Vinylchlorid (VC) zu Ethen (Eigenschaften siehe Tabellen 6 bis 8)

Anwendungsbereich: Reinigung von Wasser, kontaminiert durch DCE oder VC

Reaktorform: wahlweise Festbett-, Schwebebett-, Wirbelbettreaktor

Aufwuchsmaterial: wahlweise Kunststoff, Aluminiumoxid, Keramik, Sinterglas, Quarzsand, Bims

Prozeßführung: Einstufiger, anaerober Prozeß

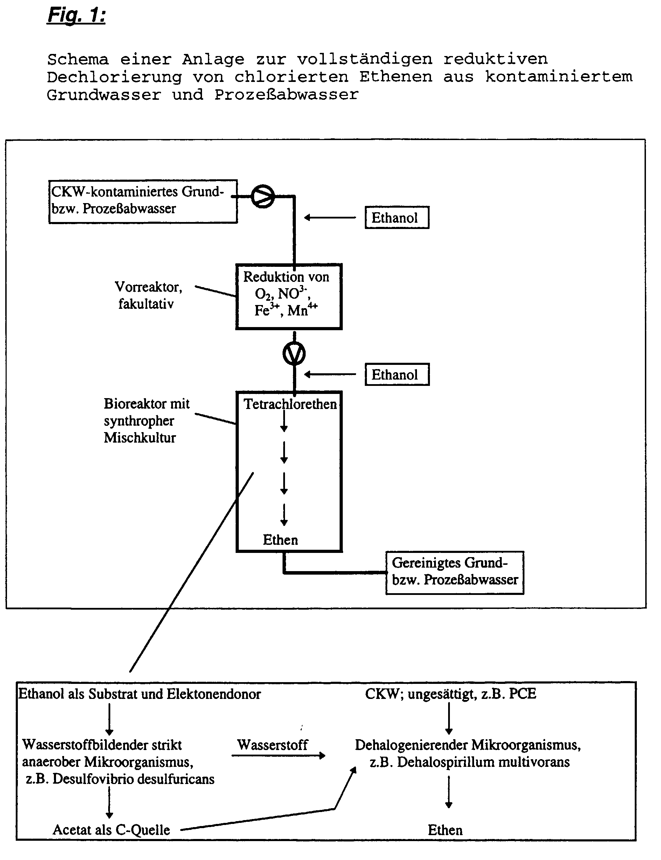

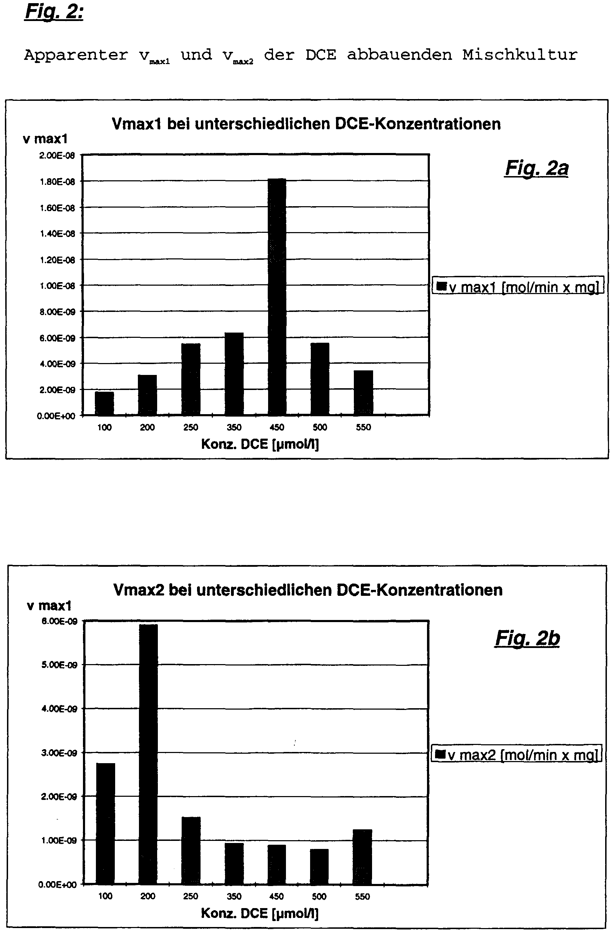

Es wurden folgende Umsatzraten der Bakterien in Batchversuchen (nur Organismus C ohne Organismus A, siehe Beispiel 7 und Fig. 2):

$$V_{max1} = 18 \text{ nmol/min/mg Zellprotein (cis-1,2-Dichlorethen} \rightarrow \text{Vinylchlorid)}$$

$$V_{max2} = 6 \text{ nmol/min/mg Zellprotein (Vinylchlorid} \rightarrow \text{Ethen)}$$

Umsatzraten der Bakterien (nur Organismus C) im Wirbelschichtreaktor (V = 0,7; Q = 50 ml/h; PCE in = 0,2 mmol/l; Ethanol in = 20 mmol/l; 20 °C):

$$V_{DCE \rightarrow Ethen} \geq 15 \ \mu\text{mol/h/l Reaktorvolumen}$$

Durch syntrophe Verfahrensweisen ergeben sich analoge Vorteile wie in Beispiel 1 (PCE-Dechlorierung) für die DCE-Dechlorierung im vorliegenden Beispiel.

**Beispiel 3:**

Syntrophe Dechlorierung von PCE-Kontaminationen in Wasser bis zum Ethen

Die Elimination von PCE-Kontaminationen in Wasser über TCE, DCE bis zum Ethen wurde als syntrophes Kombinationsverfahren der in den Beispielen 1 und 2 beschriebenen Abbaustufen (Stufe 1 = PCE → DCE, Stufe 2 = DCE → Ethen) unter folgenden Bedingungen und Variationen ausgeführt:

Syntrophe Mikroorganismen-Kombination:

A) ein wasserstoffbildendes strikt anaerobes Bakterium, das gleichzeitig Acetat als Kohlenstoffouelle für den dehalogenierenden Mikroorganismus bildet:

A1: Desulfovibrio desulfuricans
A2: Syntrophobacter wolinii
A3: Saccharolytische Clostridien, z. B. C. pasteurianum.

B) ein PCE-dehalogenierendes Bakterium (Produktion von cis-1,2-Dichlorethen (DCE) aus Tetrachlorethen (PCE):

B1: Dehalospirillum multivorans
(siehe Neumann A., Scholz-Muramatsu H., Diekert G. (1994). Tetrachloroethene metabolism of Dehalospirillum multivorans. Arch. Microbiol. 162: 295-301 oder Lit. [5])

B2: Desulfitobacterium Stamm PCE-S wie im Beispiel 4 isoliert und in Beispiel 5 charakterisiert.
(siehe Christiansen N., Ahring BK. (1996). Desulfitobacterium hafniense sp. nov., an Anaerobic, reductively dechlorinating bacterium. Int. J. Syst. Bac. 46: 442-448)

C) hochangereicherte Mischkultur aus 3 bis 4 verschiedenen Bakterienarten mit der Fähigkeit zur Dechlorierung von cis-1,2-Dichlorethen (DCE) über Vinylchlorid (VC) zu Ethen (Eigenschaften siehe Tabellen 6 bis 8)

Anwendungsbereich: Reinigung von Wasser, kontaminiert durch PCE oder TCE, DCE und VC

Reaktorform: wahlweise Festbett-, Schwebebett-, Wir belbettreaktor

Aufwuchsmaterial: wahlweise Kunststoff, Aluminiumoxid, Keramik, Sinterglas, Quarzsand, Bims

Prozeßführung: Einstufiger, anaerober Prozeß

Durch das syntrophe Kombinationsverfahren können die Verfahrensstufen 1 und 2, wie sie in den Beispielen 1 und 2 beschrieben sind, unter Ausnutzung der gegebenen Vorteile in effizienter Weise als Einstufenverfahren durchgeführt werden.

**Beispiel 4:**

Herkunft, Isolierung und Charakteristika einer Tetrachlorethen dechlorierenden Reinkultur und einer cis-1,2-Dichlorethen dechlorierenden angereicherten Mischkultur

Herkunft:

Die Untersuchungen gingen von einer Mischkultur aus, die Tetrachlorethen (PCE) vollständig zu Ethen dechlorieren konnte. Diese Kultur wurde vom Technologiezentrum Wasser, DVGW Karlsruhe zur Verfügung gestellt und aus Bodenproben der ehemaligen Mülldeponie Eppelheim angereichert. Die ehemalige Mülldeponie Eppelheim ist als Modellstandort für unterschiedliche Sanierungsverfahren bekannt und ist durch stellenweise CKW-Kontaminationen gekennzeichnet.
Die Mischkultur setzte sich aus 4 bis 5 morphologisch unterschiedlichen Zelltypen zusammen und war durch eine starke Methanogenese gekennzeichnet. Sie zeichnete sich durch ein breites Substratspektrum aus (siehe Tabelle 1).

Tabelle 1

Spektrum der genutzten Elektronendonoren der Eppelheimmischkultur
**Einfluß der Elektronen-Donoren auf die Umsatzdauer von Tetrachlorethen (Anfangskonzentration 100 µmol/l, 30°C)**

| Elektronen-Donoren | Zeitraum für 50% Umsatz [d] | Biomasseproduktion [mg/l] |
|---|---|---|
| $H_2$ | 7.0 | 0 |
| Formiat | 6.0 | 0 |
| Ethanol | 7.5 | 0 |
| Pyruvat | 4.5 | 20 |
| Glycerin | >10 | 60 |
| Glucose | >10 | >140 |

Die vollständige Dechlorierung fand auch bei Grundwasser üblichen Temperaturen statt (12 °C).

Anreicherung:

Damit die Mischkultur kontinuierlich versorgt werden konnte, wurde ein Festbettreaktor aufgebaut (Volumen: 250 ml, Elektronen-Donor: 20 mmol/l Ethanol, Elektronenakzeptor: PCE, 20 °C). Somit konnte die Kultur fortlaufend in der logarithmischen Phase gehalten werden. Der Einsatz von hohen PCE Konzentrationen (ca. 500 umol/l) bot, zusätzlich zu einer Anreichung der dechlorierenden Mikroorganismen, noch den Vorteil, daß die Methanogenese zurückgedrängt werden konnte. Bei Einsatz der Mischkultur in einem künstlichen Aquifer konnten so Probleme durch eine starke Methanogenese, insbesondere bei der Hydraulik, vermieden werden.
Um die Methanogenese zurückzudrängen, wurden auch in Batchansätzen hohe PCE-Konzentrationen verwendet, sowie der Einsatz unterschiedlicher Hemmstoffe (Monensin, Ethen, Bromethansulfonsäure) getestet. Monensin wirkte als Ionophor und führte somit zum Zusammenbruch des Na+ Gradienten, Bromethansulfonsäure ist strukturanalog zu Coenzym M der Methanbildner. Während hohe PCE-Konzentrationen und Ethen (5%ig in der Gasphase) den gewünschten Erfolg brachten, hemmte Bromethansulfonsäure die Dechlorierung von cis-1,2-Dichlorethen (DCE) auch schon in geringen Konzentrationen (0,5 mmol/l). Die mehrmalige Verwendung von Monensin war zu vermeiden, da die häufige Verwendung die Kultur im Wachstum und in der Dechlorierungfähigkeit schwächte.

Isolierung:

Um Reinkulturen zu erhalten, wurden Müller-Krempel Flaschen (V:250ml) in der Innenwandung mit Agar beschichtet (Roll-tubes), mit 1 ml 0,5 molarem PCE in Hexadecan (im Fall von DCE: 0,005 mmol/l) versetzt und unter Schutzgas-

atmosphäre (90 % $N_2$/10 % $CO_2$) mit einem Vitonstopfen verschlossen. Als Elektronen-Donoren dienten Pyruvat (40mmol/l) oder Wasserstoff (1 bar Überdruck) oder Formiat (40 mmol/l). Diese Elektronen-Donoren wurden in die entsprechende Menge Mineralmedium [5] gegeben, in den heißen Agar eingespritzt und sofort in Eiswasser blasenfrei gerollt, so daß sich eine Innenwandbeschichtung aus Agar ergab. Darauf wachsende Kolonien konnten so unter Schutzgasatmosphäre isoliert und später in Flüssigmedium kultiviert werden.
Als Inoculum diente ein Batchansatz, der über 10 Generationen hinweg mit hohen PCE Konzentrationen (ca. 500 umol/l) und 40 mmol/l Pyruvat gefüttert worden war. Nach ca. zwei Wochen wurden verschiedene Kolonien isoliert. Es konnten dann mehrere Kulturen gefunden werden, die PCE sehr schnell zu DCE abbauten, aber noch aus 2 bis 3 morphologisch unterschiedlichen Zelltypen bestanden. Die oben beschriebene Prozedur wurde daraufhin wiederholt. Dabei konnte eine PCE dechlorierende Reinkultur gewonnen werden. Sie wurde von einem Rolltube abgepickt, das als Elektronen-Donor Wasserstoff (1 bar Überdruck) enthielt; sowie Hefeextrakt als C-Quelle (20 mmol/l, 0,2 %). Obwohl in der Gasphase der Roll-tubes immer Vinylchlorid (VC) gemessen werden konnte, war es nicht möglich eine Kultur zu isolieren, die DCE abbauen konnte. Daraufhin wurden Verdünnungsreihen, ebenfalls mit Wasserstoff (1 bar Überdruck) und Hefeextrakt, angesetzt, wohingegen hier als Inoculum Biofilm aus dem oben beschriebenen Festbettreaktor verwendet wurde. Hierbei konnte eine hochangereicherte Mischkultur ohne Methanogenese mit der Fähigkeit DCE zu dechlorieren gewonnen werden. Diese Mischkultur setzte sich nunmehr aus 3 bis 4 genetisch unterschiedlichen Bakterienarten zusammen.

**Beispiel 5:**

Charakterisierung der PCE dechlorierenden Reinkultur (Desulfitobacterium Stamm PCE-S).

Test mit Antikörpern [2] auf die bis dato bekannten PCE-Dechlorierer Dehalospirillum multivorans und mit Gensonden auf Dehalobacter restrictus [1] ergaben keine positiven Ergebnisse. Um die taxonomische Herkunft des Bakteriums, vorläufig Desulfitobacterium Stamm PCE-S genannt, zu klären, wurde von der GBF Braunschweig eine 165 rNA-Analyse durchgeführt. Hierbei konnte über 99,5 % Identität mit Desulfitobacterium hafniense [3] nachgewiesen werden. Zur genauen Identifikation erfolgte eine DNA/DNA Hybridisierung durch die DSM mit Desulfitobacterium hafniense und weiteren verwandten Organismen. Die Ergebnisse sind in Tab. 2 aufgeführt.

Tabelle 2

| DNA/DNA Hybridisierung (Übereinstimmung zwischen den Bakterien-Stämmen) | | | |
|---|---|---|---|
| **DNA-DNA Hybridiserung** | **Desulfitobacterium Stamm PCE-S** | **D. hafniese** | **D. dehalogenans** |
| D.hafniense | 91,2% | --- | --- |
| D. dehalogenans | 55,4% | 37,4% | --- |
| D.species strain PCE 1 | 58,7% | 53,4% | 94% |

Der G+C-Gehalt im Vergleich zu Adenin und Thymin beträgt bei allen Organismen ca. 47 %.
Zur Morphologie des Desulfitobacteriums Stamm PCE-S können aus der nachfolgenden Tabelle 3 Angaben (im Vergleich zu D. hafniense) entnommen werden.

Tabelle 3

| Morphologische Merkmale von Desulfitobacterium Stamm PCE-S und D. hafniense | | |
|---|---|---|
| **Merkmale** | **Desulfitobacterium Stamm PCE S** | **D.hafniense** |
| Sporenbildung | + | + |
| gekrümmtes Stäbchen, Größe: bis 5 μm lang, 0,6 - 0,7 μm breit | + | + |
| beweglich | + | + |
| grampositiv | + | + |
| Wachstum auf Pepton | - | - |

Tabelle 3 (fortgesetzt)

| Morphologische Merkmale von Desulfitobacterium Stamm PCE-S und D. hafniense | | |
|---|---|---|
| **Merkmale** | **Desulfitobacterium Stamm PCE S** | **D.hafniense** |
| Wachstum auf Trypton | gering | + |
| Wachstum auf Nährgelatine | - | - |
| Wachstum bei 37 °C | + | + |
| Wachstum auf Pyruvat und/oder Hefe und Nutzung als Elektronendonor zu Dechlorierung | + | + |

Als weitere Elektronendonoren können noch Formiat und Wasserstoff genutzt werden, allerdings ist hier kein Wachstum, auch nicht unter Zugabe von Acetat, möglich. Die Dechlorierung wird am stärksten durch Formiat und Wasserstoff unterstützt, gefolgt von Pyruvat. Hefeextrakt als Elektronen-Donor ergibt einen nur sehr langsamen Dechlorierungsprozeß.
Als Elektronenakzeptoren konnten bis jetzt Fumarat und Sulfit gefunden werden. Sulfit hemmt die PCE-Dechlorierung, wohingegen Fumarat die Dechlorierung nur verlangsamt.

**Beispiel 6:**

Das Abbauverhalten gegenüber CKWs und eventuelle die Hemmung der PCE-Dechlorierung durch andere CKWs sind in den Tabellen 4 und 5 aufgeführt.

Tabelle 4

| Dechlorierungsverhalten von Desulfitobacterium Stamm PCE-S und Desulfitobacterium hafniense | | |
|---|---|---|
| **Abbau von** | **Desulfitobacterium PCE-S** | **D. hafniense** |
| Tetrachlorethen | + | - |
| Trichlorethen | + | ? |
| Dichlorethen | - | ? |
| 3-Chlor-4-Hydroxyphenylacetat | - | + |
| Tetrachlormethan | - | ? |
| Trichlormethan | - | ? |
| Dichlormethan | - | ? |
| 1,1,1 Trichlorethan | - | ? |
| 1,1 Dichlorethan | - | ? |
| 1,2 Dichlorethan | - | ? |

Tabelle 5

| Hemmung der PCE-Dechlorierung bei Desulfitobacterium Stamm PCE-S durch andere CKWs | |
|---|---|
| CKW (ca. 700µM) | Hemmwirkung |
| Tetrachlormethan | hemmt völlig, auch schon bei 100 µmol/l |

Tabelle 5 (fortgesetzt)

| Hemmung der PCE-Dechlorierung bei Desulfitobacterium Stamm PCE-S durch andere CKWs | |
|---|---|
| Trichlormethan | der Umsatz bleibt bei der Bildung von TCE stehen |
| Dichlormethan | - |
| 1,1,1-Trichlorethan | - |
| 1,1-Dichlorethan | - |
| 1,2-Dichlorethan | - |
| 1,1-Dichlor(1)propen | - |
| 2,3-Dichlor(1)propen | - |
| 1,3-trans- Dichlor(1)propen | - |
| 1,3-Dichlorpropanol | - |

Da dieses Bakterium aufgrund der Ergebnisse der DNA/DNA Hybridisierung und der Nutzung von Sulfit als Elektronenakzeptor in die Gattung der Desulfitobakterien eingeordnet werden kann, wird es Desulfitobacterium Stamm PCE-S genannt (das "S" beschreibt den Herkunftsort der Isolierung, Stuttgart).

**Beispiel 7:**

Charakterisierung der DCE-abbauenden angereicherten Mischkultur:

Zur Dechlorierung von DCE können viele unterschiedliche Elektronendonoren durch die angereicherte Mischkultur genutzt werden, allerdings ergeben sich unterschiedliche Dechlorierungsgeschwindigkeiten:

Qualität der Elektronendonoren im Hinblick auf die Dechlorierungsgeschwindigkeit von DCE:
H2/Ac = Pyruvat = Ethanol = Formiat/Acetat/Hefe = Formiat/Acetat >Glycerin = Glucose = Methanol.

Qualität der Elektronendonoren im Hinblick auf die Dechlorierungsgeschwindigkeit von VC:
H2/Acetat > Pyruvat >> Ethanol > Formiat/Acetat/Hefe > Formiat/Acetat ≥ Glycerin > Glucose.

Qualität der Elektronendonoren im Hinblick auf die Ethenbildung:
H2/Acetat = Ethanol > Pyruvat > Formiat/Acetat/Hefe > Glycerin = Methanol > Formiat/Acetat > Glucose

Das Wachstum und der Umsatz zu weiteren Cosubstraten ist in den Tabellen 6 und 7 aufgeführt.

Tabelle 6

| Wachstum der DCE-abbauenden angereicherten Mischkultur mit verschiedenen Cosubstraten | | |
|---|---|---|
| **Cosubstrat** | **Konzentration** | **Wachstum** |
| Formiat/Acetat | 20 mM/10 mM | n.n. |
| $H_2$/Acetat | 1 bar/10 mM | n.n. |
| Formiat/Acetat/Hefe | 20 mM/10 mM/0,1 % | + |
| Pyruvat | 20 mM | + |
| Ethanol | 20 mM | + |
| Glycerin | 20 mM | n.n. |
| Glucose | 10 mM | n.n. |
| Methanol | 20 mM | n.n. |

Tabelle 7

| Umsatz verschiedener Cosubstrate durch die DCE abbauende angereicherte Mischkultur | | |
|---|---|---|
| **Cosubstrat** | **Konzentration** | **Produktbildung** |
| Formiat/Acetat | 20 mM/10 mM | n.n. |
| $H_2$/Acetat | 1 bar/10 mM | n.n. |
| Formiat/Acetat/Hefe | 20mM/10 mM/0,1 % | n.n. |
| Pyruvat | 20mM | Acetat > Propionat |
| Ethanol | 20 mM | Propionat ≥ Acetat, |
| Glycerin | 20 mM | Propionat |
| Glucose | 10 mM | Acetat > Succinat, |
| Methanol | 20 mM | Propionat und Acetat |

Als Elektronenakzeptoren können, neben DCE und VC, Fumarat, Nitrat, Sulfat in geringem Maße, Polysulfid und Fe (+III) dienen. Die Dechlorierung wird aber nur durch Fe (+III) und Polysulfid gehemmt.
Sowohl DCE als auch VC hemmen oberhalb bestimmter Konzentrationen die Dechlorierung. Das Optimum der DCE Konzentration für die DCE Dechlorierung beträgt 450 µmol/l. Der apparente Vmax1 hierbei beträgt 18 nmol/(min x mg) Zellprotein. Für den weiteren Umsatz von VC zu Ethen liegt das Optimum der DCE Konzentration bei 200 µmol/l, der apparente Vmax2 liegt hier bei 6 nmol/ (min x mg) Zellprotein (siehe Fig. 2).
Das weitere Verhalten der DCE-Mischkultur gegenüber anderen CKWs ist in Tabelle 8 beschrieben.

Tabelle 8

| Hemmung der DCE-Dechlorierung durch andere CKWs | |
|---|---|
| **CKW (ca. 200µM)** | **Hemmwirkung auf die Dechlorierung** |
| Tetrachlormethan | hemmt völlig |
| Trichlormethan | hemmt völlig |
| Dichlormethan | hemmt VC Umsatz völlig, DCE Umsatz nicht |
| 1,1,1-Trichlorethan | hemmt völlig |
| 1,1-Dichlorethan | verlangsamt den VC Umsatz |
| 1,2-Dichlorethan | hemmt nicht, wird vermutlich biotisch umgesetzt, das Endprodukt ist noch nicht bekannt. |

**Beispiel 8:**

Wachstum und Dechlorierung von Dehalospirillum multivorans in Co-Kultur mit anderen Bakterien

Untersuchungen zeigten, daß Dehalospirillum multivorans in Co-Kultur mit anderen Bakterien wachsen und dechlorieren kann, welche ihm die Reduktionsäquivalente liefern, die er zur Dechlorierung von Tetrachlorethen (PCE) und Trichlorethen (TCE) (und somit zur Gewinnung von Energie) benötigt. Diese Untersuchungen wurden vorgenommen, um den Einsatz billiger, technisch relevanter Substrate zu testen, welche von D. multivorans nicht direkt verwertet werden können, aber von anderen Organismen umgesetzt werden. Die freiwerdenden Elektronen und die entstehenden Produkte kann D. multivorans zum Abbau der CKW und zum Wachstum nutzen.
Das syntrophe anaerobe Bakterium Syntrophobacter wolinii verwertet Propionsäure als Substrat und wandelt sie zu Essigsäure, $CO_2$ und Wasserstoff um.
Als syntrophes Bakterium ist S. wolinii auf ein zweites Bakterium angewiesen, das die freiwerdenden Elektronen verwertet. D. multivorans, welches Propionsäure nur sehr schlecht verwerten kann, nutzt die Elektronen von S. wolinii zur Dechlorierung von PCE und TCE und das entstehende Acetat als C-Quelle.
Die folgende Tabelle 9 zeigt die Ergebnisse von Batch-Versuchen, die bei 30 °C durchgeführt wurden.

Tabelle 9

| | **15 mmol/l Propionat, 10 mmol/l PCE, 0,2% Hefeextrakt D. multivorans, S. wolinii** | **10 mmol/l PCE, $H_2$ 5mmol/l Acetat D. multivorans** |
|---|---|---|
| Dechlorierungsraten [nmol $Cl^-$/(min×mg Protein)] | 423 | 401 |
| Gebildete Biomasse [mg Protein/mmol $Cl^-$] | 0,817 | 0,879 |
| (Die Angaben "mg Protein" beziehen sich auf mg Zellprotein von D. multivorans; nmol bzw. mmol $Cl^-$ ist die Menge Chlorid, die aus PCE bezogen auf 1 mg Zellprotein pro 1 Minute freigesetzt wurde) | | |

Die Ergebnisse zeigen, daß die syntrophe Co-Kultur aus Syntrophobacter wolinii und Dehalospirillum multivorans sehr effektiv die chlorierten Kohlenwasserstoffe abbaut. Die Dechlorierungsraten sind so hoch wie in einer Reinkultur von D. multivorans bei optimaler Versorgung mit Wasserstoff als Elektronen-Donor und Acetat als C-Quelle. In diesen Versuchen wurde Hefeextrakt für das Wachstum von S. wolinii zugegeben. Allerdings ist in Mischkulturen in der Regel keine Zugabe von Hefeextrakt erforderlich, da die Bakterien sich gegenseitig versorgen.
Insbesondere Ethanol kommt als selektives und billiges Substrat für eine technische Anwendung in Frage. Desulfovibrio desulfuricans, ein anaerobes sulfatreduzierendes Bakterium, oxidiert Ethanol zu Acetat und überträgt die freiwerdenden Elektronen auf Sulfat. Dazu benötigt es keinen Hefeextrakt.
Sowohl in Gegenwart als auch in Abwesenheit von Sulfat konnte D. multivorans die freiwerdenden Elektronen zur Dechlorierung nutzen. Die gefundenen Dechlorierungsraten sind in Gegenwart von Sulfat etwa so hoch wie ohne Sulfat, aber der Ethanolverbrauch steigt in Gegenwart von Sulfat an. Hieraus folgt, daß der Sulfatreduzierer mehr Ethanol umsetzen kann, wenn zusätzlich Sulfat als Elektronenakzeptor vorhanden ist. Die Dechlorierung wird von der Sulfat-Anwesenheit nicht beeinträchtigt.
Die folgende Tabelle 10 zeigt die Dechlorierungsraten, die in dieser Mischkultur in Batch-Experimenten bei 30 °C erzielt wurden. Die Vorteile verschiedener strikt wasserstoffbildender Mikroorganismen sind in Tabelle 12 angegeben.

Tabelle 10

| Dechlorierungsraten syntropher Mischkulturen | | | |
|---|---|---|---|
| | **10 mmol/l Ethanol, 10 mmol/l PCE, 15 mmol/l Sulfat D. multivorans, D. desulfricans** | **10 mmol/l Ethanol, 10 mmol/l PCE D. multivorans D. desulfuricans** | **10 mmol/l PCE, $H_2$ 5 mmol/l Acetat D. multivorans** |
| Dechlorierungsraten [nmol Cl-(min×mg Protein)] | 900 | 800 | 1000 |

Tabelle 11

| Eigenschaften verschiedener Wasserstoffproduzierender Bakterien (Organismus A) im Hinblick auf die syntrophe Dechlorierung von Tetrachlorethen | | | |
|---|---|---|---|
| **Bakterium** | **Substrat** | **Produkte** | **$H_2$-Freisetzung pro Mol Substrat** |
| A1 Fakultativ Syntrophe z. B. Desulfovibrio desulfuricans | Ethanol | Acetat, $H_2$ | 4 Mol |
| A2 Obligat Syntrophe z. B. Syntrophobacter wolinii | Propionsäure | Acetat, $H_2$ | 3 Mol |

Tabelle 11 (fortgesetzt)

| Eigenschaften verschiedener Wasserstoffproduzierender Bakterien (Organismus A) im Hinblick auf die syntrophe Dechlorierung von Tetrachlorethen | | | |
|---|---|---|---|
| **Bakterium** | **Substrat** | **Produkte** | **$H_2$-Freisetzung pro Mol Substrat** |
| A3 Saccharolytische Clostridien z. B. C. pasteurianum | Glucose, Pyruvat | Butyrat, Acetat, Ethanol, $CO_2$, $H_2$ | 4 Mol |

Tabelle 12

| Vorteile verschiedener Wasserstoff-produzierender Organismen im Hinblick auf eine syntrophe Dechlorierung | |
|---|---|
| **Bakterium** | **Vorteil** |
| A1 Fakultativ Syntrophe Desulfovibrio desulfuricans (fakultativ syntroph) | • langsames Wachstum auf Ethanol;<br>• $H_2$-Freisetzung erlaubt effektive Dechlorierung von PCE zu Ethen;<br>• Bildung von Acetat liefert Kohlenstoff für das Wachstum von D. multivorans und ev. auch der DCE-Dechlorierer;<br>• Ethanol ist ein sehr selektives Substrat, das von nur wenigen Anaerobiem genutzt wird. |
| A2 Obligat Syntrophe Syntrophobacter wolinii (obligat syntroph) | • langsames Wachstum auf Propionsäure;<br>• $H_2$-Freisetzung erlaubt effektive Dechlorierung von PCE zu Ethen;<br>• Bildung von Acetat liefert Kohlenstoff für das Wachstum von D. multivorans und ev. auch der DCE-Dechlorierer;<br>• Propionsaure ist ein sehr selektives Substrat, das von nur wenigen Anaerobiern genutzt wird. |
| A3 Saccharolytische Clostridien z. B. C. pasteurianum | • Freisetzungsrate von $H_2$ ist viel größer als bei den Organismen A1 und A2 da der Substratumsatz rascher ist → möglicherweise raschere Dechlorierung der chlorierten Ethene;<br>• Dechlorierung von $\gamma$-HCH durch Clostridien wie z. B. C. pasteurianum (Jagnow et al., 1977) |

**Beispiel 9:**

Klonierung und Sequenzierung der Gene der Tetrachlorethen-Dehalogenase (PCE-RD); siehe auch Figuren 3 bis 5.

1. Reinigung der PCE-RD aus Dehalospirillum multivorans siehe Literatur [8].

2. Verdauung des gereinigten Proteins mit Trypsin, Auftrennung der dadurch entstandenen Oligopeptide durch präparative HPLC (siehe Literatur [9], Aminosäure-Sequenzierung (durchgeführt vom Fraunhofer Institut für Grenzflächenund Bioverfahrenstechnik, Stuttgart) von vier der isolierten Oligopeptide mit einer Länge von jeweils 9 bis 22 Aminosäuren.

3. Synthese von insgesamt 4 Oligonukleotiden aus der Sequenz von 2 der sequenzierten Oligopeptide (Peptid 1 und 4 der unter II. angegebenen Aminosäuresequenz). Pro Oligopeptid wurden zwei Oligonukleotide mit invers komplementären Sequenzen synthetisiert. Die Synthese wurde von der Firma Genset (Paris, Frankreich) durchgeführt. Dabei wurde für die jeweils letzte Position der Basentripletts, die für Aminosäuren mit degeneriertem Code kodieren, Inosin bzw. Basenmischungen eingesetzt. Die folgenden Oligonukleotide wurden verwendet :

| | |
|---|---|
| Aus Peptid 1 | Oligonukleotid 1: GGI GAG GTI AAG CCI TGG TT |
| | Oligonukleotid 2 : AAC CAI GGY TTI ACY TCN CC |
| Aus Peptid 4 | Oligonukleotid 3: ATI ACI GAG GTI TGG GAY GG |
| | Oligonukleotid 4: GTC CCA IAC YTC IGT DAT RTT |

4. Durchführung der PCR (siehe Literatur [10] ) mit den angegebenen Oligonukleotiden unter Verwendung genomischer Dehalospirillum multivorans-DNA als Template.

5. Klonierung und Ansequenzierung des mit dem Primerpaar Oligonukleotid 2 und 3 entstanden PCR-Produktes. Das PCR-Produkt wurde in das Plasmid pBluescript® II KS (+) der Firma Stratagene, Heidelberg, ligiert und in E. coli transformiert (Literatur [10]). Das entstandene Plasmid wurde als pW3 bezeichnet.

6. Herstellung einer DIG-markierten Gensonde aus dem klonierten PCR-Produkt unter Verwendung des "DIG DNA Labelling and Detection Kit Nonradioactive" der Firma Boehringer, Mannheim, nach Angaben des Herstellers.

7. Herstellung einer angereicherten Genbank mit Hilfe der Gensonde (Literatur [10]).

8. Klonierung eines 6 kb EcoRI-Fragmentes (Literatur [10]), das das vollständige PCE-Dehalogenase-Gen enthielt.

9. Sequenzierung des 6 kB-Fragmentes mit Hilfe synthetisierter Oligonukleotide und durch Subklonierung (Literatur [10]) unter Verwendung des "PRISM Ready Reaction Dye Deoxy Terminator AmpliTaq" bzw. "PRISM Ready Reaction Dye Deoxy Terminator AmpliTaq FS" Kit der Firma Perkin Elmer ABI, Weiterstadt, nach Angaben des Herstellers. Die Sequenzierung erfolgte unter Verwendung eines automatischen Sequenzierers.

<u>Zur weiteren Erläuterung dieses Beispiels dienen die Figuren 3 bis 5:</u>

Der im Plasmid pW3 enthaltene Anteil des PCE-Dehalogenase-Gens ist fett und unterstrichen dargestellt. Diese Sequenz eignet sich durch ihre Länge und dadurch bedingte Spezifität als Gensonde für die Detektion der PCE-Dehalogenase aus Dehalospirillum multivorans. Auch andere hinreichend lange Bereiche des Gens sind als Gensonde brauchbar. Die verwendete Sonde (komplette Sequenz) reagierte spezifisch gegen Dehalospirillum multivorans. Es wurden weder gegen einen weiteren PCE-Dechlorierer (Desulfitobacterium spp. Stamm PCE-S) noch gegen E. coli Kreuzreaktionen beobachtet.
Direkt im Anschluß an das PCE-Dehalogenase-Gen mit einer Überlappung von 4 Basen findet sich ein offener Leserahmen (ORF), der gemäß der Sequenz und der daraus folgenden Aminosäurezusammensetzung für ein hydrophobes Protein kodiert. Daß der Transkriptionsstop erst nach dieser Sequenz in Form einer "Hairpin"-Struktur erfolgt, zeigt, daß dieser Abschnitt zur PCE-Dehalogenase gehört. Es wird angenommen, daß das durch diesen Abschnitt kodierte Protein einen "Membrananker" der PCE-Dehalogenase darstellt.

**Patentansprüche**

**1.** Verfahren zur mikrobiologischen Reinigung von Wässern, die mit chlorierten Ethenen und/oder chlorierten Propenen kontaminiert sind, dadurch gekennzeichnet, daß man die mit chlorierten Ethenen und/oder chlorierten Propenen belasteten Wässer, vorzugsweise Grundwässer oder Prozeßabwässer, unter Zusatz einer ausreichenden Menge einer Elektronendonor-Substanz über einen Bioreaktor leitet, der eine definierte, an einen Träger immobilisierte, syntrophe Mischkultur enthält, die aus wenigstens einem dehalogenierenden Bakterium und wenigstens einem wasserstoffbildenden strikt anaeroben Mikroorganismus besteht, wobei der wasserstoffbildende strikt anaerobe Mikroorganismus vorzugsweise gleichzeitig eine C-Quelle für das dehalogenierende Bakterium liefert.

**2.** Verfahren nach Anspruch 1, dadurch gekennzeichnet, daß der wasserstoffbildende strikt anaerobe Mikroorganismus ein sulfidogenes Bakterium, insbesondere ein sulfatreduzierendes Bakterium ist.

**3.** Verfahren nach Anspruch 2, dadurch gekennzeichnet, daß das sulfatreduzierende Bakterium Desulfovibrio desulfuricans ist.

4. Verfahren nach einem der Ansprüche 1 bis 3, vorzugsweise nach Anspruch 3, dadurch gekennzeichnet, daß als Elektronendonor-Substanz Ethanol zugesetzt wird.

5. Verfahren nach Anspruch 1, dadurch gekennzeichnet, daß das dehalogenierende Bakterium ("Dehalogenierer") ein Dehalospirillum multivorans, ein Desulfitobacterium Stamm PCE-S, oder eine hochangereicherte anaerobe Mischkultur ist, die Dichlorethen (DCE) zu Ethen reduziert.

6. Verfahren nach einem der Ansprüche 1 bis 5, dadurch gekennzeichnet, daß der Bioreaktor ein Schlaufenreaktor mit Wirbelbett ist.

7. Syntrophe Mischkultur bestehend aus a) einem Dehalogenierer, der aus der Gruppe Dehalospirillum multivorans, Desulfitobacterium Stamm PCE-S und einer hochangereicherten anaeroben Mischkultur, die Dichlorethen zu Ethen abbaut, ausgewählt ist; und b) dem wasserstoffbildenden strikt anaeroben Mikroorganismus Desulfovibrio desulfuricans.

8. Desulfitobacterium Stamm PCE-S, welches erhältlich ist durch Isolierung als Reinkultur aus einer angereicherten Mischkultur aus einer Bodenprobe der ehemaligen Mülldeponie Eppelheim, indem man in Gegenwart einer Elektronendonor-Substanz und von PCE als Substrat mit der Hungate-Technik Kolonien auf Agar-Agar zieht, wobei das Inoculum zuvor in einem Batchverfahren über einige Generationen mit hohen PCE-Konzentrationen und Pyruvat gefüttert wurde, und wobei das Desulfitobacterium Stamm PCE-S durch die Eigenschaften bzw. Merkmale in den Tabellen 1 und 2 charakterisiert ist.

9. Verfahren zum Nachweis und/oder zur Kontrolle potentiell an einem Untersuchungs- oder Behandlungsort vorhandener Dehalogenase-Aktivität, vorzugsweise in einem PCE-kontaminierten Boden- und/oder Grundwasserbereich oder in einem Bioreaktor mit immobilisiertem Zellmaterial, dadurch gekennzeichnet, daß man genetisches Material aus dem zu untersuchenden Boden- und/oder Grundwasserbereich oder aus dem zu kontrollierenden Bioreaktor isoliert und in konventioneller Weise mit einer zu der in Fig. 4 angegebenen, wenigstens 80 % homologen DNA-Sequenz für das Dehalogenase-Gen aus Dehalospirillum multivorans oder mit wenigstens einer 0,2 kB-großen, Teilsequenz davon in an sich üblicher Weise hybridisiert und man nachfolgend den im positiven Falle gebildeten, hybridisierten DNA-Strang in an sich üblicher Weise weiterverarbeitet und identifiziert.

10. DNA-Sequenz mit wenigstens 80 % Homologie zu der in Fig. 4 angegebenen DNA-Sequenz für das Dehalogenase-Gen aus Dehalospirillum multivorans.

11. Verwendung einer DNA-Sequenz gemäß Anspruch 10 oder einer 0,2 kB-großen Teilsequenz davon als Gensonde zum Nachweis von und/oder zur von Kontrolle potentiell an einem Untersuchungs- oder Behandlungsort vorhandenen Dehalogenase-Gen enthaltender Bakterien, vorzugsweise in einem PCE-kontaminierten Boden- und/oder Grundwasserbereich oder in einem Bioreaktor mit immobilisiertem Zellmaterial.

## *Fig. 1:*

Schema einer Anlage zur vollständigen reduktiven Dechlorierung von chlorierten Ethenen aus kontaminiertem Grundwasser und Prozeßabwasser

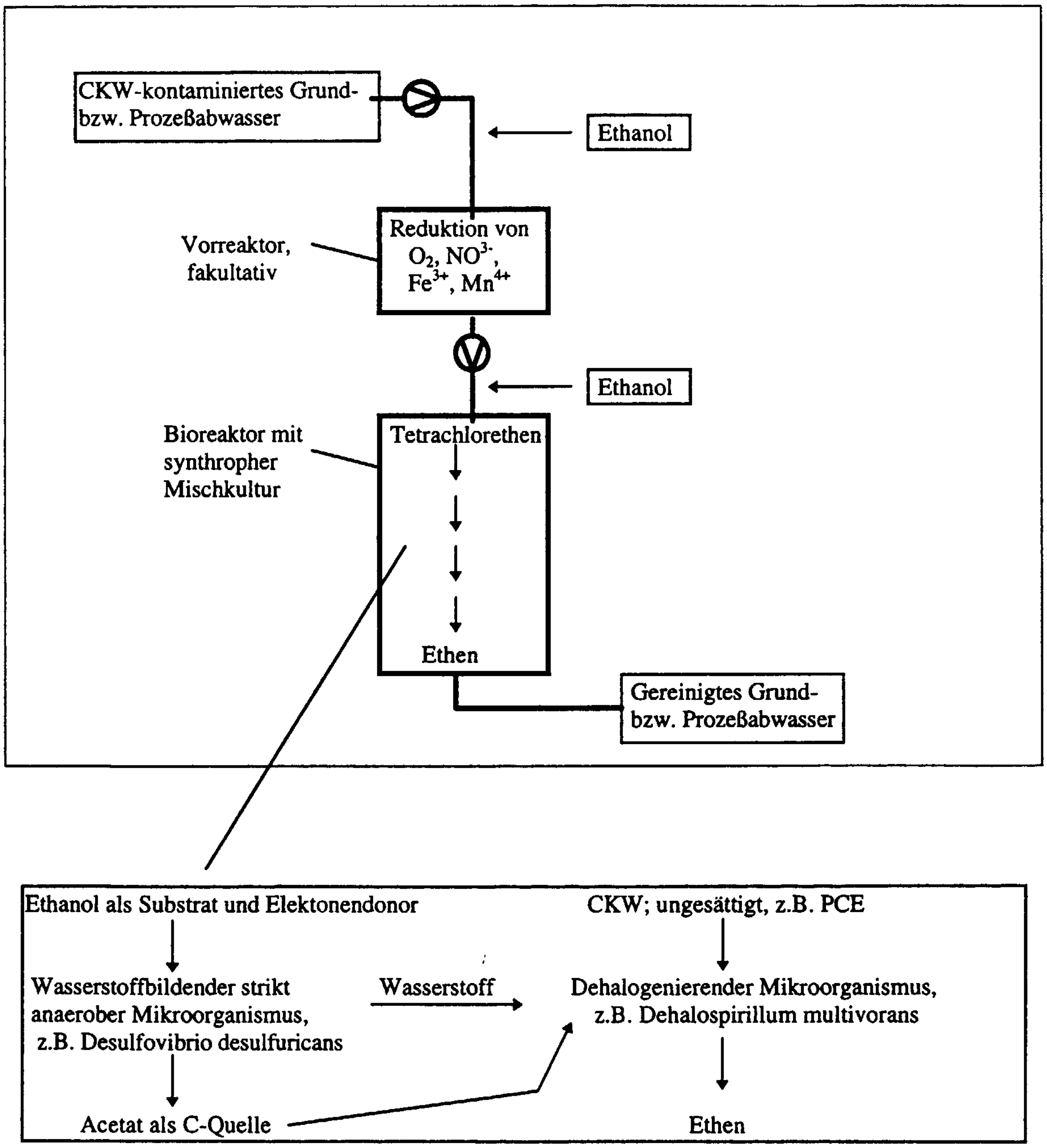

*Fig. 2:*

Apparenter $v_{max1}$ und $v_{max2}$ der DCE abbauenden Mischkultur

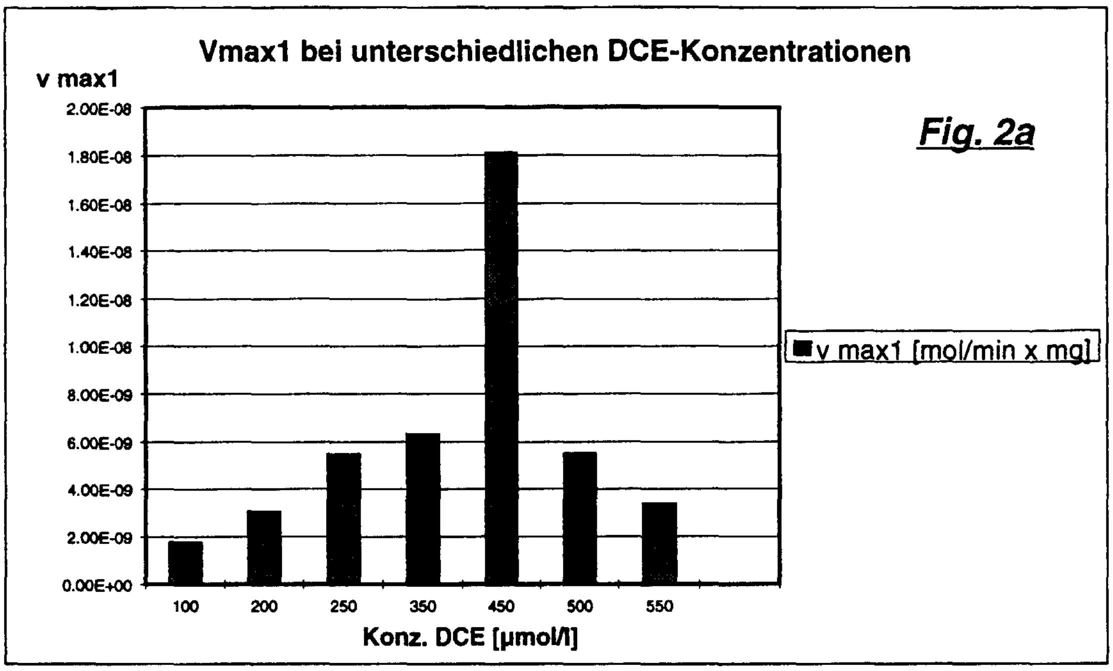


*Fig. 2a*

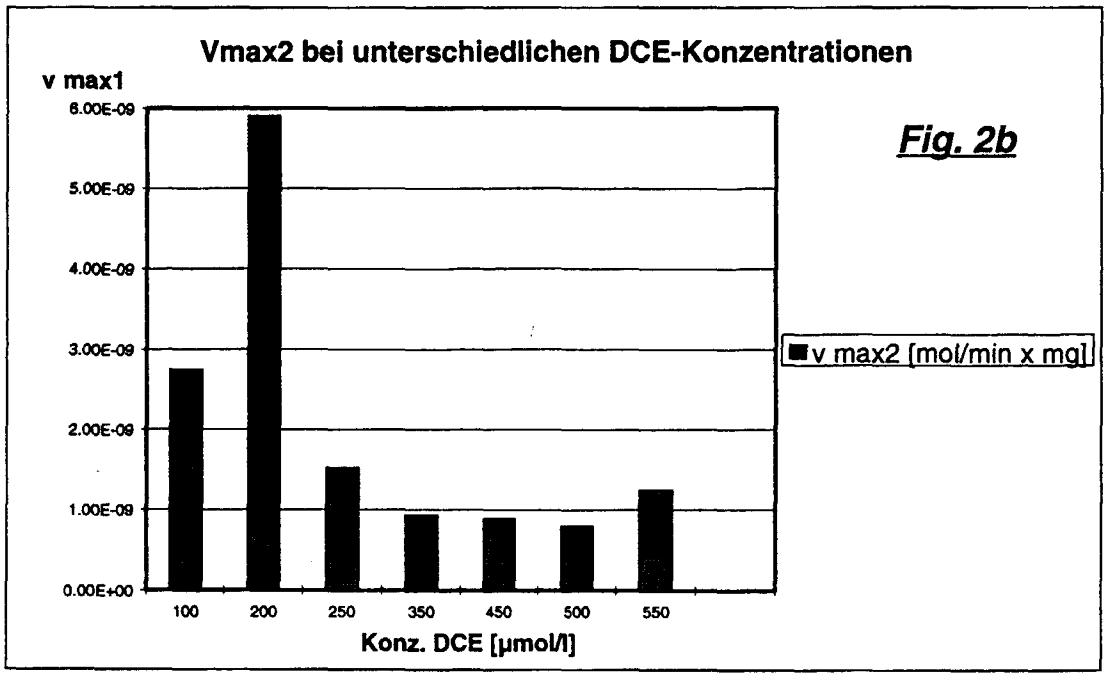


*Fig. 2b*

## *Fig. 3:*

Aminosäuresequenz der PCE-Dehalogenase (PCE-RD) aus *Dehalospirillum multivorans*

Die fett und unterstrichen markierten Bereiche entsprechen den nach tryptischem Verdau isolierten Oligopeptiden 1-4:

Peptid 1, Aminosäure Nr. 88-99

Peptid 2, Aminosäure Nr. 127-146

Peptid 3, Aminosäure Nr. 204-225

Peptid 4, Aminosäure Nr. 468-476

5 10 15 20 25 30

| | | | | |

1 M E K K K K P E L S R R D F G K L I I G G G A A A T I A P F

31 G V P G A N A A E K E K N A A E I R Q Q F A M T A G S P I I

61 V N D K L E R Y A E V R T A F T H P T S F F K P N Y K **G E V**

91 **K P W F L S A Y D** E K V R Q I E N G E N G P K M K A K N V G

121 E A R A G R **A L E A A G W T L D I N Y G N I Y P N R** F F M L

151 W S G E T M T N T Q L W A P V G L D R R P P D T T D P V E L

181 T N Y V K F A A R M A G A D L V G V A R L N R **N W V Y S E A**

211 **V T I P A D V P Y E Q S L H K** E I E K P I V F K D V P L P I

241 E T D D E L I I P N T C E N V I V A G I A M N R E M M Q T A

271 P N S M A C A T T A F C Y S R M C M F D M W L C Q F I R Y M

301 G Y Y A I P S C N G V G Q S V A F A V E A G L G Q A S R M G

331 A C I T P E F G P N V R L T K V F T N M P L V P D K P I D F

361 G V T E F C E T C K K C A R E C P S K A I T E G P R T F E G

391 R S I H N Q S G K L Q W Q N D Y N K C L G Y W P E S G G Y C

421 G V C V A V C P F T K G N I W I H D G V E W L I D N T R F L

451 D P L M L G M D D A L G Y G A K R **N I T E V W D G K** I N T Y

481 G L D A D H F R D T V S F R K D R V K K S

## *Fig. 4:*

DNA-Basensequenz des PCE-Dehalogenase-Gens (PCE-RD-Gen)

```
        10         20         30         40         50         60
         |          |          |          |          |          |
                                ATGGAAAAG AAAAAAAAGC CTGAACTCTC
AAGAAGAGAT TTTGGTAAGT TGATTATCGG AGGTGGAGCT GCAGCAACGA TAGCTCCATT
TGGTGTACCA GGTGCAAATG CAGCTGAAAA AGAGAAAAAT GCAGCTGAAA TTCGTCAACA
ATTTGCAATG ACTGCAGGTT CTCCCATCAT AGTTAACGAC AAATTGGAAA GATATGCTGA
AGTACGAACA GCGTTTACTC ATCCAACCTC ATTTTTTAAA CCAAACTACA AAGGTGAAGT
GAAACCTTGG TTTTTATCAG CATATGATGA GAAAGTACGT CAGATTGAAA ATGGTGAAAA
TGGTCCTAAG ATGAAAGCTA AAAATGTAGG AGAAGCTAGA GCAGGTCGTG CACTTGAAGC
TGCTGGATGG ACCTTAGATA TTAACTATGG AAACATTTAT CCAAATAGAT TTTTTATGTT
ATGGTCTGGT GAGACTATGA CTAACACCCA ATTGTGGGCA CCAGTAGGGT TAGATAGAAG
ACCTCCAGAT ACAACTGATC CTGTGGAACT TACAAATTAT GTAAAATTTG CTGCACGTAT
GGCAGGTGCT GATTTGGTAG GTGTTGCAAG ATTAAATCGT AACTGGGTTT ATTCTGAGGC
AGTAACTATA CCAGCTGACG TACCTTATGA ACAATCTTTG CATAAAGAGA TTGAAAAGCC
AATCGTTTTC AAAGATGTTC CACTACCAAT AGAAACTGAT GATGAATTAA TTATCCCTAA
TACTTGTGAA AATGTTATCG TTGCAGGTAT CGCTATGAAC CGCGAAATGA TGCAAACAGC
TCCTAACAGT ATGGCATGTG CAACAACAGC ATTTTGTTAT TCACGTATGT GTATGTTCGA
TATGTGGTTA TGCCAGTTTA TTCGTTATAT GGGTTACTAT GCAATACCAA GCTGTAATGG
TGTTGGACAA TCAGTTGCCT TTGCTGTTGA AGCAGGTTTA GGACAAGCTA GTCGTATGGG
TGCTTGTATT ACTCCTGAAT TTGGACCAAA CGTAAGACTT ACAAAAGTCT TTACAAATAT
GCCTTTAGTT CCAGATAAGC CTATCGACTT TGGAGTAACA GAATTTTGTG AAACATGTAA
AAAATGTGCA CGTGAGTGTC CTTCAAAAGC AATTACTGAA GGTCCAAGAA CTTTTGAAGG
ACGAAGTATT CATAATCAAT CAGGTAAATT ACAGTGGCAA AATGACTATA ATAAATGCTT
AGGTTATTGG CCGGAATCTG GTGGATATTG TGGCGTATGT GTAGCTGTTT GCCCCTTTAC
AAAAGGCAAT ATTTGGATTC ATGATGGCGT TGAATGGCTT ATTGATAACA CAAGATTCTT
AGACCCATTA ATGCTTGGTA TGGATGATGC ATTGGGCTAT GGTGCAAAAC GAAATATTAC
AGAAGTTTGG GATGGAAAGA TTAATACATA TGGTCTAGAC GCAGACCATT TTAGAGACAC
TGTAAGCTTT AGAAAGGATA GGGTTAAAAA ATCA
```

## *Fig. 5a:*

Sequenz des "Membranankers" der PCE-RD

```
        10         20         30         40         50         60
         |          |          |          |          |          |
                                  ATGAAAT TATTAAATAT TTTAAATTAT
AAAGCGCTTG GTTTGAGTTT ACTTTATTTG GCATTGATTT TGGTTACTTT CCAAATTTCA
ATGGGAAGTT ATGGAGAGGA TGAGCCACAA GCTGGTAGTA TATTAATGGT TGCTGGATTG
ATTTTTTCGA TCATCGGCGT TTTTCTTTTA ATGAAGTTCA AGCCAACATA TATTTTATGG
GAAAAGCTTA AGCGT
```

## *Fig. 5b:*

Sequenz des "Membranankers" der PCE-RD aus Fig. 5a
übersetzt in die Aminosäuresequenz

```
          5         10         15         20         25         30
          |          |          |          |          |          |
 1 M K L L N I L N Y K A L G L S L L Y L A L I L V T F Q I S M
31 G S Y G E D E P Q A G S I L M V A G L I F S I I G V F L L M
61 K F K P T Y I L W E K L K R
```

*Fig. 6:* SEQUENZPROTOKOLL

(1) ALLGEMEINE ANGABEN:

(i) ANMELDER:
(A) NAME: Solvay Deutschland GmbH
(B) STRASSE: Hans-Boeckler Allee 20
(C) ORT: Hannover
(E) LAND: Deutschland
(F) POSTLEITZAHL: D-30171
(G) TELEFON: (0511) 857-0
(H) TELEFAX: (0511) 282126

(ii) BEZEICHNUNG DER ERFINDUNG:
Verfahren zur biologischen Behandlung von Grund- oder Prozessabwaessern zur Entfernung von halogenierten, ungesaettigten Kohlenwasserstoffen

(iii) ANZAHL DER SEQUENZEN: 4

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(vi) DATEN DER URANMELDUNG:
(A) ANMELDENUMMER: DE 19710010.4
(B) ANMELDETAG: 12-MAR-1997

(2) ANGABEN ZU SEQ ID NO: 1:

(i) SEQUENZKENNZEICHEN:
(A) LÄNGE: 501 Aminosäuren
(B) ART: Aminosäure
(C) STRANGFORM: Einzelstrang
(D) TOPOLOGIE: linear

(ii) ART DES MOLEKÜLS: Protein

(vi) URSPRÜNLICHE HERKUNFT:
(A) ORGANISMUS: Dehalospirillum multivorans

(ix) MERKMAL:
(A) NAME/SCHLÜSSEL: Peptide
(B) LAGE:88..99
(D) SONSTIGE ANGABEN:/label= Oligopeptid
/note= "Nach tryptischem Verdau isoliertes Oligopeptid"

(ix) MERKMAL:
(A) NAME/SCHLÜSSEL: Peptide
(B) LAGE:127..146
(D) SONSTIGE ANGABEN:/label= Oligopeptid
/note= "Nach tryptischem Verdau isoliertes Oligopeptid"

(ix) MERKMAL:
(A) NAME/SCHLÜSSEL: Peptide
(B) LAGE:204..225
(D) SONSTIGE ANGABEN:/label= Oligopeptid
/note= "Nach tryptischem Verdau isoliertes Oligopeptid"

(ix) MERKMAL:
(A) NAME/SCHLÜSSEL: Peptide
(B) LAGE:468..476
(D) SONSTIGE ANGABEN:/label= Oligopeptid
/note= "Nach tryptischem Verdau isoliertes Oligopeptid"

(xi) SEQUENZBESCHREIBUNG: SEQ ID NO: 1:

Met Glu Lys Lys Lys Lys Pro Glu Leu Ser Arg Arg Asp Phe Gly Lys
1 5 10 15

Leu Ile Ile Gly Gly Gly Ala Ala Ala Thr Ile Ala Pro Phe Gly Val
20 25 30

Pro Gly Ala Asn Ala Ala Glu Lys Glu Lys Asn Ala Ala Glu Ile Arg
35 40 45

Gln Gln Phe Ala Met Thr Ala Gly Ser Pro Ile Ile Val Asn Asp Lys
50 55 60

Leu Glu Arg Tyr Ala Glu Val Arg Thr Ala Phe Thr His Pro Thr Ser
65 70 75 80

Phe Phe Lys Pro Asn Tyr Lys Gly Glu Val Lys Pro Trp Phe Leu Ser
85 90 95

Ala Tyr Asp Glu Lys Val Arg Gln Ile Glu Asn Gly Glu Asn Gly Pro
100 105 110

Lys Met Lys Ala Lys Asn Val Gly Glu Ala Arg Ala Gly Arg Ala Leu
115 120 125

Glu Ala Ala Gly Trp Thr Leu Asp Ile Asn Tyr Gly Asn Ile Tyr Pro
130 135 140

Asn Arg Phe Phe Met Leu Trp Ser Gly Glu Thr Met Thr Asn Thr Gln
145 150 155 160

Leu Trp Ala Pro Val Gly Leu Asp Arg Arg Pro Pro Asp Thr Thr Asp
165 170 175

Pro Val Glu Leu Thr Asn Tyr Val Lys Phe Ala Ala Arg Met Ala Gly
180 185 190

Ala Asp Leu Val Gly Val Ala Arg Leu Asn Arg Asn Trp Val Tyr Ser
195 200 205

Glu Ala Val Thr Ile Pro Ala Asp Val Pro Tyr Glu Gln Ser Leu His
210 215 220

Lys Glu Ile Glu Lys Pro Ile Val Phe Lys Asp Val Pro Leu Pro Ile
225 230 235 240

Glu Thr Asp Asp Glu Leu Ile Ile Pro Asn Thr Cys Glu Asn Val Ile
245 250 255

Val Ala Gly Ile Ala Met Asn Arg Glu Met Met Gln Thr Ala Pro Asn
260 265 270

```
Ser Met Ala Cys Ala Thr Thr Ala Phe Cys Tyr Ser Arg Met Cys Met
        275                 280                 285

Phe Asp Met Trp Leu Cys Gln Phe Ile Arg Tyr Met Gly Tyr Tyr Ala
    290                 295                 300

Ile Pro Ser Cys Asn Gly Val Gly Gln Ser Val Ala Phe Ala Val Glu
305                 310                 315                 320

Ala Gly Leu Gly Gln Ala Ser Arg Met Gly Ala Cys Ile Thr Pro Glu
                325                 330                 335

Phe Gly Pro Asn Val Arg Leu Thr Lys Val Phe Thr Asn Met Pro Leu
            340                 345                 350

Val Pro Asp Lys Pro Ile Asp Phe Gly Val Thr Glu Phe Cys Glu Thr
        355                 360                 365

Cys Lys Lys Cys Ala Arg Glu Cys Pro Ser Lys Ala Ile Thr Glu Gly
    370                 375                 380

Pro Arg Thr Phe Glu Gly Arg Ser Ile His Asn Gln Ser Gly Lys Leu
385                 390                 395                 400

Gln Trp Gln Asn Asp Tyr Asn Lys Cys Leu Gly Tyr Trp Pro Glu Ser
                405                 410                 415

Gly Gly Tyr Cys Gly Val Cys Val Ala Val Cys Pro Phe Thr Lys Gly
            420                 425                 430

Asn Ile Trp Ile His Asp Gly Val Glu Trp Leu Ile Asp Asn Thr Arg
        435                 440                 445

Phe Leu Asp Pro Leu Met Leu Gly Met Asp Asp Ala Leu Gly Tyr Gly
    450                 455                 460

Ala Lys Arg Asn Ile Thr Glu Val Trp Asp Gly Lys Ile Asn Thr Tyr
465                 470                 475                 480

Gly Leu Asp Ala Asp His Phe Arg Asp Thr Val Ser Phe Arg Lys Asp
                485                 490                 495

Arg Val Lys Lys Ser
            500
```

(2) ANGABEN ZU SEQ ID NO: 2:

(i) SEQUENZKENNZEICHEN:
(A) LÄNGE: 1503 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Dehalospirillum multivorans

(xi) SEQUENZBESCHREIBUNG: SEQ ID NO: 2:

```
ATGGAAAAGA AAAAAAAGCC TGAACTCTCA AGAAGAGATT TTGGTAAGTT GATTATCGGA     60
GGTGGAGCTG CAGCAACGAT AGCTCCATTT GGTGTACCAG GTGCAAATGC AGCTGAAAAA    120
GAGAAAAATG CAGCTGAAAT TCGTCAACAA TTTGCAATGA CTGCAGGTTC TCCCATCATA    180
GTTAACGACA AATTGGAAAG ATATGCTGAA GTACGAACAG CGTTTACTCA TCCAACCTCA    240
TTTTTTAAAC CAAACTACAA AGGTGAAGTG AAACCTTGGT TTTTATCAGC ATATGATGAG    300
AAAGTACGTC AGATTGAAAA TGGTGAAAAT GGTCCTAAGA TGAAAGCTAA AAATGTAGGA    360
GAAGCTAGAG CAGGTCGTGC ACTTGAAGCT GCTGGATGGA CCTTAGATAT TAACTATGGA    420
AACATTTATC CAAATAGATT TTTTATGTTA TGGTCTGGTG AGACTATGAC TAACACCCAA    480
TTGTGGGCAC CAGTAGGGTT AGATAGAAGA CCTCCAGATA CAACTGATCC TGTGGAACTT    540
ACAAATTATG TAAAATTTGC TGCACGTATG GCAGGTGCTG ATTTGGTAGG TGTTGCAAGA    600
TTAAATCGTA ACTGGGTTTA TTCTGAGGCA GTAACTATAC CAGCTGACGT ACCTTATGAA    660
CAATCTTTGC ATAAAGAGAT TGAAAAGCCA ATCGTTTTCA AAGATGTTCC ACTACCAATA    720
GAAACTGATG ATGAATTAAT TATCCCTAAT ACTTGTGAAA ATGTTATCGT TGCAGGTATC    780
GCTATGAACC GCGAAATGAT GCAAACAGCT CCTAACAGTA TGGCATGTGC AACAACAGCA    840
TTTTGTTATT CACGTATGTG TATGTTCGAT ATGTGGTTAT GCCAGTTTAT TCGTTATATG    900
GGTTACTATG CAATACCAAG CTGTAATGGT GTTGGACAAT CAGTTGCCTT TGCTGTTGAA    960
GCAGGTTTAG GACAAGCTAG TCGTATGGGT GCTTGTATTA CTCCTGAATT TGGACCAAAC   1020
GTAAGACTTA CAAAAGTCTT TACAAATATG CCTTTAGTTC CAGATAAGCC TATCGACTTT   1080
GGAGTAACAG AATTTTGTGA AACATGTAAA AAATGTGCAC GTGAGTGTCC TTCAAAAGCA   1140
ATTACTGAAG GTCCAAGAAC TTTTGAAGGA CGAAGTATTC ATAATCAATC AGGTAAATTA   1200
CAGTGGCAAA ATGACTATAA TAAATGCTTA GGTTATTGGC CGGAATCTGG TGGATATTGT   1260
GGCGTATGTG TAGCTGTTTG CCCCTTTACA AAAGGCAATA TTTGGATTCA TGATGGCGTT   1320
GAATGGCTTA TTGATAACAC AAGATTCTTA GACCCATTAA TGCTTGGTAT GGATGATGCA   1380
TTGGGCTATG GTGCAAAACG AAATATTACA GAAGTTTGGG ATGGAAAGAT TAATACATAT   1440
GGTCTAGACG CAGACCATTT TAGAGACACT GTAAGCTTTA GAAAGGATAG GGTTAAAAAA   1500
TCA                                                                 1503
```

(2) ANGABEN ZU SEQ ID NO: 3:

(i) SEQUENZKENNZEICHEN:
- (A) LÄNGE: 222 Basenpaare
- (B) ART: Nucleotid
- (C) STRANGFORM: Einzelstrang
- (D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Dehalospirillum multivorans

(xi) SEQUENZBESCHREIBUNG: SEQ ID NO: 3:

```
ATGAAATTAT TAAATATTTT AAATTATAAA GCGCTTGGTT TGAGTTTACT TTATTTGGCA      60

TTGATTTTGG TTACTTTCCA AATTTCAATG GGAAGTTATG GAGAGGATGA GCCACAAGCT     120

GGTAGTATAT TAATGGTTGC TGGATTGATT TTTTCGATCA TCGGCGTTTT TCTTTTAATG     180

AAGTTCAAGC CAACATATAT TTTATGGGAA AAGCTTAAGC GT                        222
```

(2) ANGABEN ZU SEQ ID NO: 4:

(i) SEQUENZKENNZEICHEN:
(A) LÄNGE: 74 Aminosäuren
(B) ART: Aminosäure
(C) STRANGFORM: Einzelstrang
(D) TOPOLOGIE: linear

(ii) ART DES MOLEKÜLS: Peptid

(vi) URSPRÜNLICHE HERKUNFT:
(A) ORGANISMUS: Dehalospirillum multivorans

(xi) SEQUENZBESCHREIBUNG: SEQ ID NO: 4:

```
Met Lys Leu Leu Asn Ile Leu Asn Tyr Lys Ala Leu Gly Leu Ser Leu
1               5                   10                  15

Leu Tyr Leu Ala Leu Ile Leu Val Thr Phe Gln Ile Ser Met Gly Ser
            20                  25                  30

Tyr Gly Glu Asp Glu Pro Gln Ala Gly Ser Ile Leu Met Val Ala Gly
        35                  40                  45

Leu Ile Phe Ser Ile Ile Gly Val Phe Leu Leu Met Lys Phe Lys Pro
    50                  55                  60

Thr Tyr Ile Leu Trp Glu Lys Leu Lys Arg
65                  70
```